# EUROPEAN PATENT SPECIFICATION

(11) **EP 0 801 058 B1**
(45) Date of publication and mention of the grant of the patent: **10.10.2001**
(21) Application number: 97302272.6
(22) Date of filing: 02.04.1997
(51) Int. Cl.: C07D 207/34, C07D 207/36, C07D 207/42, A01N 43/36

(54) **Arylthio, -sulfinyl and -sulfonyl pyrrole insecticidal agents**
Arylthio, -sulfinyl und -sulfonyl-Pyrrol mit insektizider Wirkung
Pyrroles substitués par une groupe arylthio, -sulfinyl et -sulfonyl ayant un activité insecticide

(30) Priority: 10.04.1996 US 631763
(43) Date of publication of application: 15.10.1997
(73) Proprietor: American Cyanamid Company, Madison, New Jersey 07940-0874 (US)
(72) Inventor: Barnes, Keith Douglas, Newtown, Pennsylvania 18940 (US); Diehl, Robert Eugene, Yardley, Pennsylvania 19067 (US); Trotto, Susan Hensen, Yardley, Pennsylvania 19067 (US); Hu, Yulin, Plainsboro, New Jersey 08536 (US)
(74) Representative: Langfinger, Klaus Dieter, Dr.

(56) References cited:
- EP-A- 0 549 866
- EP-A- 0 591 806
- EP-A- 0 708 090

## Description

### BACKGROUND OF THE INVENTION

Insects destroy growing and harvested crops. In the United States, agronomic crops must compete with thousands of insect species. In particular, tobacco budworms and southern armyworms are especially devastating to crops.

Tobacco budworms cause tremendous economic losses in agronomic crops. In particular, budworms devastate cotton crops by feeding on green bolls. Control of budworms is complicated by their resistance to many common insecticides, including organophosphates, carbamates and pyrethroids. Also, budworm larvae are difficult to control with currently available insecticides once they reach the third instar.

In spite of the commercial insecticides available today, damage to crops, both growing and harvested, caused by insects still occurs. Accordingly, there is ongoing research to create new and more effective insecticides.

Certain pyrrole compounds are known to possess insecticidal and acaricidal activity (see, e.g. United States Patent Numbers 5,010,098; 5,281,719; 5,306,827 and 5,310,938; EP-A2-372982; and CA-A1-2,076,937). However, none of the pyrrole compounds disclosed in those patents and patent applications are within the scope of the present invention.

It is, therefore, an object of the present invention to provide compounds which are highly effective for controlling insects.

It is also an object of the present invention to provide a method for controlling insects.

It is a further object of this invention to provide a method for protecting growing plants from attack by insects.

### SUMMARY OF THE INVENTION

The present invention describes arylthio, -sulfinyl and -sulfonyl pyrrole compounds which are useful for the control of insects and the protection of plants from attack by insects.

The arylthio, -sulfinyl and -sulfonyl pyrrole compounds of the present invention have the structural formula I 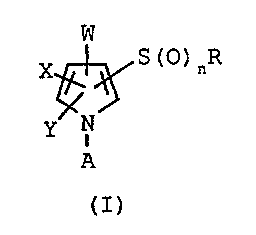 wherein
R and X are each independently phenyl optionally substituted with any combination of from one to five halogen, NO₂, CN, C₁-C₄alkyl, C₁-C₄haloalkyl, C₁-C₄alkoxy or C₁-C₄haloalkoxy groups;
n is an integer of 0, 1 or 2;
W is halogen, CN, NO₂ or C₁-C₄haloalkyl;
Y is hydrogen, halogen or C₁-C₄haloalkyl;
A is hydrogen, CN, C(O)R₁, CHR₂NHC(O)R₃, CH₂SQ, CHR₄OC(O) (CR₅R₆)ₘQ₁,
   C₁-C₆alkyl optionally substituted with
   one to three halogen atoms,
   one tri(C₁-C₄alkyl)silyl,
   one hydroxy,
   one cyano,
   one or two C₁-C₄alkoxy groups optionally substituted with one to three halogen atoms,
   one C₁-C₄alkylthio,
   one phenyl optionally substituted with one to three substituents the same or different selected from halogen, C₁-C₄alkyl and C₁-C₄alkoxy,
   one phenoxy group optionally substituted with one to three substituents the same or different selected from halogen, C₁-C₄alkyl and C₁-C₄alkoxy,
   one benzyloxy group optionally substituted on the phenyl ring with one to three substituents the same or different selected from halogen, C₁-C₄alkyl and C₁-C₄alkoxy,
   one C₁-C₆alkylcarbonyloxy group optionally substituted with one to three halogen atoms,
   one C₂-C₆alkenylcarbonyloxy group optionally substituted with one to three halogen atoms,
   one phenylcarbonyloxy group optionally substituted with one to three substituents the same or different selected from halogen, C₁-C₄alkyl, and C₁-C₄alkoxy,
   one C₁-C₆alkoxycarbonyl group optionally substituted with one to three halogen atoms or one to three C₁-C₄alkoxy groups, or
   one benzylcarbonyloxy group optionally substituted on the phenyl ring with one to three substituents the same or different selected from halogen, C₁-C₄alkyl and C₁-C₄alkoxy,
   C₃-C₆alkenyl optionally substituted with one to three halogen atoms or one phenyl group, or
   C₃-C₆alkynyl optionally substituted with one to three halogen atoms or one phenyl group;
R₁ is C₁-C₆alkyl or C₃-C₆cycloalkyl each optionally substituted with
   one to three halogen atoms,
   one hydroxy,
   one cyano,
   one or two C₁-C₄alkoxy groups optionally substituted with one to three halogen atoms,
   one C₁-C₄alkylthio,
   one phenyl group optionally substituted with one to three substituents the same or different selected from halogen, C₁-C₄alkyl and C₁-C₄alkoxy,
   one phenoxy group optionally substituted with one to three substituents the same or different selected from halogen, C₁-C₄alkyl and C₁-C₄alkoxy,
   one benzyloxy group optionally substituted on the phenyl ring with one to three substituents the same or different selected from halogen, C₁-C₄alkyl and C₁-C₄alkoxy,
   one C₁-C₆alkylcarbonyloxy group optionally substituted with one to three halogen atoms,
   one C₂-C₆alkenylcarbonyloxy group optionally substituted with one to three halogen atoms,
   one phenylcarbonyloxy group optionally substituted with one to three substituents the same or different selected from halogen, C₁-C₄alkyl and C₁-C₄alkoxy,
   one C₁-C₆alkoxycarbonyl group optionally substituted with one to three halogen atoms or one to three C₁-C₄alkoxy groups, or
   one benzyloxycarbonyl group optionally substituted on the phenyl ring with one to three substituents the same or different selected from halogen, C₁-C₄alkyl and C₁-C₄alkoxy,

   C₂-C₆alkenyl optionally substituted with one to three halogen atoms or one phenyl group,
   C₃-C₆alkynyl optionally substituted with one to three halogen atoms or one phenyl group,
   phenyl optionally substituted with one or more substituents the same or different selected from halogen, C₁-C₄alkyl, C₁-C₄alkoxy, phenoxy, C₁-C₄alkylthio, tri(C₁-C₄alkyl)silyl, C₁-C₄alkylsulfinyl, C₁-C₄alkylsulfonyl, CN, NO₂ and CF₃,
   phenoxy optionally substituted with one or more substituents the same or different selected from halogen, C₁-C₄alkyl, C₁-C₄alkoxy, C₁-C₄alkylthio, tri(C₁-C₄alkyl)silyl, C₁-C₄alkylsulfinyl, C₁-C₄alkylsulfonyl, CN, NO₂ and CF₃,
   1- or 2-naphthyl,
   2-, 3- or 4-pyridyl optionally substituted with one to three halogen atoms,
   C₁-C₆alkoxy optionally substituted with one to three halogen atoms, or
   C₂-C₆alkenyloxy optionally substituted with one to three halogen atoms;
R₂ is hydrogen or C₁-C₄alkyl;
R₃ is C₁-C₆alkyl optionally substituted with one to three halogen atoms,
   phenyl optionally substituted with one to three substituents the same or different selected from halogen, CN, NO₂, C₁-C₄alkyl, C₁-C₄alkoxy, and CF₃,
   2- or 3-thienyl, or
   2- or 3-furyl;
Q is 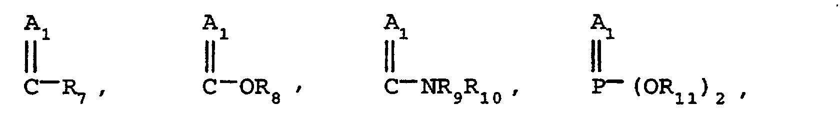 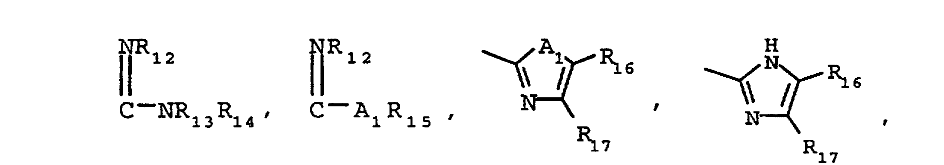 CN,
   C₁-C₆alkyl optionally substituted with one or more halogen atoms, CN groups or phenyl groups, or
   phenyl optionally substituted with one or more substituents the same or different selected from halogen, C₁-C₄alkyl, C₁-C₄alkoxy, CN, NO₂, CF₃ and NR₁₈R₁₉;
A₁ is O or S;
R₇ is C₁-C₆alkyl or phenyl;
R₈ is C₁-C₆alkyl;
R₉ and R₁₀ are each independently hydrogen, C₁-C₆alkyl or may be taken together with the atom to which they are attached to form a 5- to 7-membered heterocyclic ring;
R₁₁ is C₁-C₄alkyl;
R₁₂ is hydrogen, C₁-C₄alkyl or may be taken together with either R₁₃ or R₁₅ and the atoms to which they are attached to form a 5- to 7-membered heterocyclic ring optionally substituted with one or two C₁-C₄alkyl groups;
R₁₃ and R₁₄ are each independently hydrogen or C₁-C₄alkyl;
R₁₅ is C₁₋C₄ alkyl or when taken together with R₁₂ and the atoms to which they are attached may form a 5- to 7- membered heterocyclic ring optionally substituted with one or two C₁-C₄alkyl groups;
R₁₆ and R₁₇ are each independently hydrogen or C₁-C₄alkyl or when taken together may form a ring wherein R₁₆R₁₇ is represented by -CH=CH-CH=CH-;
R₁₈ and R₁₉ are each independently hydrogen or C₁-C₄alkyl;
R₄ is hydrogen or C₁-C₄alkyl;
R₅ and R₆ are each independently hydrogen,
   C₁-C₆alkyl optionally substituted with one or more halogen atoms,
   C₁-C₆alkoxy optionally substituted with one or more halogen atoms,
   C₁-C₆alkylthio optionally substituted with one or more halogen atoms, or
   phenyl optionally substituted with one or more halogen atoms,
      NO₂ groups;
      CN groups,
      C₁-C₄alkyl groups optionally substituted with one or more halogen atoms, or
      C₁-C₄alkoxy groups optionally substituted with one or more halogen atoms, or
   when R₅ and R₆ are taken together with the atom to which they are attached may form a C₃-C₆cycloalkyl group optionally substituted with one to three C₁-C₄alkyl groups, C₂-C₆alkenyl groups or phenyl groups, or R₅ or R₆ may be taken together with R₂₀ and the atoms to which they are attached to form a 4- to 7-membered heterocyclic ring;
m is an integer of 0, 1, 2, 3 or 4;
Q₁ is A₂R₂₀, 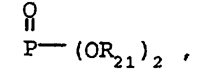 NR₂₂R₂₃, CR₂₄R₂₅C(O)R₂₆, or
   C₃-C₆cycloalkyl optionally substituted with one or more C₁-C₆alkyl groups, C₂-C₆alkenyl groups, or
   phenyl groups optionally substituted with one or more substituents the same or different selected from halogen atoms,
      NO₂ groups,
      CN groups,
      C₁-C₄alkyl groups optionally substituted with one or more halogen atoms, or
      C₁-C₄alkoxy groups optionally substituted with one or more halogen atoms;
A₂ is O or S(O)ₚ;
p is an integer of 0, 1 or 2;
R₂₀ is hydrogen,
   C₁-C₆alkyl,
   C₂-C₆alkenyl,
   C₂-C₆alkynyl,
   phenyl optionally substituted with one or more substituents the same or different selected from halogen atoms,
      NO₂ groups,
      CN groups,
      C₁-C₄alkyl groups optionally substituted with one or more halogen atoms,
      C₁-C₄alkoxy groups optionally substituted with one or more halogen atoms,
   C(O)R₂₇ provided p is 0,
   C(O)R₂₈ provided p is 0,
   (CH₂CH₂O)_{q}R₂₇, or 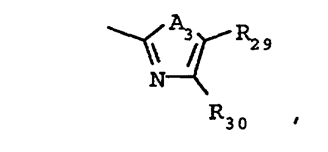 or
   R₂₀ may be taken together with either R₅ or R₆ and the atoms to which they are attached to form a 4- to 7- membered heterocyclic ring;
A₃ is O or S;
R₂₇ is C₁-C₆alkyl,
   C₂-C₆alkenyl,
   C₂-C₆alkynyl, or
   phenyl optionally substituted with one or more substituents the same or different selected from halogen atoms,
      NO₂ groups,
      CN groups,
      C₁-C₄alkyl groups optionally substituted with one or more halogen atoms, or
      C₁-C₄alkoxy groups optionally substituted with one or more halogen atoms;
q is an integer of 1, 2 or 3;
R₂₈ is OR₃₁ or NR₃₂R₃₃;
R₃₁ is C₁-C₆alkyl or
   phenyl optionally substituted with one or more substituents the same or different selected from halogen atoms,
   NO₂ groups,
   CN groups,
   C₁-C₄alkyl groups optionally substituted with one or more halogen atoms, or
   C₁-C₄alkoxy groups optionally substituted with one or more halogen atoms;
R₃₂ and R₃₃ are each independently hydrogen or C₁-C₄alkyl;
R₂₉ and R₃₀ are each independently hydrogen or C₁-C₄alkyl, or when taken together may form a ring wherein R₂₉R₃₀ is represented by -CH=CH-CH=CH-;
R₂₁ is C₁-C₄alkyl;
R₂₂ is hydrogen,
   C₁-C₆alkyl,
   C₂-C₆alkenyl,
   C₂-C₆alkynyl, or
      phenyl optionally substituted with one or more substituents the same or different selected from halogen atoms,
      NO₂ groups,
      CN groups,
      C₁-C₄alkyl groups optionally substituted with one or more halogen atoms, or
      C₁-C₄alkoxy groups optionally substituted with one or more halogen atoms, or
   R₂₂ may be taken together with either R₅ or R₆ and the atoms to which they are attached to form a 4- to 7- membered heterocyclic ring;
R₂₃ is hydrogen,
   C₁-C₆alkyl,
   C₂-C₆alkenyl,
   C₂-C₆alkynyl,
      phenyl optionally substituted with one or more substituents the same or different selected from halogen atoms,
      NO₂ groups,
      CN groups,
      C₁-C₄alkyl groups optionally substituted with one or more halogen atoms, or
      C₁-C₄alkoxy groups optionally substituted with one or more halogen atoms,
   C(A₄)R₃₄,
   CN,
   SO₂R₃₅, or
   C(O)CHR₃₆NHR₃₇;
A₄ is O or S;
R₃₄ is OR₃₈, CO₂R₃₈, NR₃₉R₄₀,
   C₁-C₆alkyl optionally substituted with one to three halogen atoms,
   C₂-C₆alkenyl,
   C₂-C₆alkynyl, or
   phenyl optionally substituted with one or more substituents the same or different selected from halogen atoms,
      NO₂ groups,
      CN groups,
      C₁-C₄alkyl groups optionally substituted with one or more halogen atoms, or
      C₁-C₄alkoxy groups optionally substituted with one or more halogen atoms;
R₃₈ is C₁-C₆alkyl optionally substituted with one phenyl group, or
   phenyl optionally substituted with one or more substituents the same or different selected from halogen atoms,
   NO₂ groups,
   CN groups,
   C₁-C₄alkyl groups optionally substituted with one or more halogen atoms, or
   C₁-C₄alkoxy groups optionally substituted with one or more halogen atoms;
R₃₉ and R₄₀ are each independently hydrogen or C₁-C₄alkyl; R₃₅ is NR₄₁R₄₂,
   C₁-C₆alkyl,
   C₂-C₆alkenyl,
   C₂-C₆alkynyl, or
   phenyl optionally substituted with one or more substituents the same or different selected from halogen atoms,
      NO₂ groups,
      CN groups,
      C₁-C₄alkyl groups optionally substituted with one or more halogen atoms, or
      C₁-C₄alkoxy groups optionally substituted with one or more halogen atoms;
R₄₁ and R₄₂ are each independently hydrogen or C₁-C₄alkyl;
R₃₆ is hydrogen,
   C₁-C₄alkyl optionally substituted with
   one hydroxy group,
   one SR₄₃ group,
   one C(O)NH₂ group,
   one NH₂ group,
   one NHC(=NH)NH₂ group,
   one CO₂H group,
   one phenyl group optionally substituted with one hydroxy group,
   one 3-indolyl group or
   one 4-imidazolyl group;
R₄₃ is hydrogen or C₁-C₄alkyl;
R₃₇ is C(A₄)R₄₄;
R₄₄ is C₁-C₆alkyl optionally substituted with one or more halogen atoms,
   C₂-C₆alkoxyalkyl,
   C₁-C₆alkylthio,
   phenyl optionally substituted with one or more substituents the same or different selected from
      halogen atoms,
      NO₂ groups,
      CN groups,
      C₁-C₄alkyl groups optionally substituted with one or more halogen atoms, or
      C₁-C₄alkoxy groups optionally substituted with one or more halogen atoms,
   OR₃₈,
   CO₂R₃₈ or
   NR₃₉R₄₀;

R₂₄ and R₂₅ are each independently hydrogen,
   C₁-C₆alkyl optionally substituted with one or more halogen atoms,
   C₁-C₆alkoxy optionally substituted with one or more halogen atoms,
   C₁-C₆alkylthio optionally substituted with one or more halogen atoms,
   phenyl optionally substituted with one or more substituents the same or different selected from halogen atoms,
      CN groups,
      NO₂ groups,
      C₁-C₄alkyl groups optionally substituted with one or more halogen atoms, or
      C₁-C₄alkoxy groups optionally substituted with one or more halogen atoms, or
   when R₂₄ and R₂₅ are taken together with the atom to which they are attached may form a C₃-C₆cycloalkyl group optionally substituted with one to three C₁-C₄alkyl groups, C₂-C₆alkenyl groups or phenyl groups;
R₂₆ is OR₄₅, NR₄₁R₄₂, C₁-C₄alkyl or
   phenyl optionally substituted with one or more substituents the same or different selected from halogen atoms,
   CN groups,
   NO₂ groups,
   C₁-C₄alkyl groups optionally substituted with one or more halogen atoms, or
   C₁-C₄alkoxy groups optionally substituted with one or more halogen atoms; and
R₄₅ is C₁-C₄alkyl or
   phenyl optionally substituted with one or more substituents the same or different selected from halogen atoms,
   CN groups,
   NO₂ groups,
   C₁-C₄alkyl groups optionally substituted with one or more halogen atoms, or
   C₁-C₄alkoxy groups optionally substituted with one or more halogen atoms.

This invention also relates to compositions containing those compounds and methods for using those compounds and compositions. Advantageously, it has been found that the arylthio, -sulfinyl and -sulfonyl pyrrole compounds of the present invention, and compositions containing them, are useful for the control of insects and the protection of plants from attack by insects. The compounds of the present invention are especially useful for the control of tobacco budworms and southern armyworms.

### DETAILED DESCRIPTION OF THE INVENTION

The present invention provides a method for controlling insects which comprises contacting said insects, their breeding grounds, food supply or habitat with an insecticidally effective amount of a formula I, arylthio, -sulfinyl or -sulfonyl pyrrole compound.

The present invention also provides a method for protecting growing plants from attack by insects which comprises applying to the foliage of said plants or to the soil or water in which they are growing an insecticidally effective amount of a formula I, arylthio, -sulfinyl or -sulfonyl pyrrole compound.

The arylthio, -sulfinyl and -sulfonyl pyrrole compounds of the present invention have the structural formula I 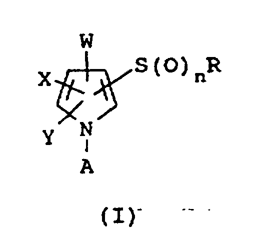 wherein A, R, W, X, Y and n are as described hereinabove for formula I.

Preferred formula I arylthio, -sulfinyl and -sulfonyl pyrrole compounds of the present invention are those wherein
R and X are each independently phenyl optionally substituted with any combination of from one to three halogen, NO₂, CN, C₁-C₄alkyl, C₁-C₄haloalkyl, C₁-C₄alkoxy or C₁-C₄haloalkoxy groups;
n is an integer of 0, 1 or 2;
W is Cl, Br, CN, NO₂ or C₁-C₄haloalkyl;
Y is hydrogen, Cl, Br or C₁-C₄haloalkyl;
A is hydrogen, CN, C(O)R₁ or
   C₁-C₄alkyl optionally substituted with
   one to three halogen atoms,
   one cyano,
   one C₁-C₄alkoxy group,
   one C₁-C₆alkylcarbonyloxy group,
   one phenylcarbonyloxy group optionally substituted with one to three halogen atoms or one C₁-C₄alkyl group, or
   one benzylcarbonyloxy group; and
R₁ is phenyl optionally substituted with one to three halogen atoms, one or two C₁-C₄alkyl groups, one or two C₁-C₄alkoxy groups, one CN group, one NO₂ group or one CF₃ group.

More preferred insecticidal agents of the present invention are those having the structural formula II 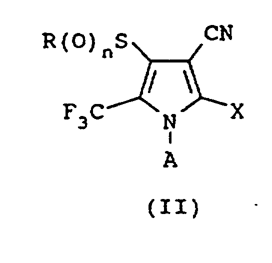 wherein
R and X are each independently phenyl optionally substituted with any combination of from one to three halogen, NO₂ or CF₃ groups;
n is an integer of 0, 1 or 2; and
A is hydrogen or C₁-C₄alkyl substituted with one , C₁-C₄alkoxy group.

Another group of more preferred insecticidal agents of this invention are those having the structural formula III 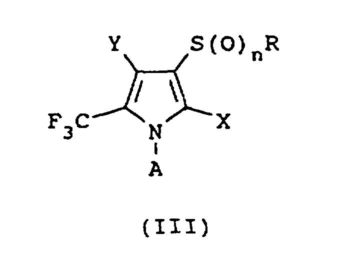 wherein
R and X are each independently phenyl optionally substituted with any combination of from one to three halogen, NO₂ or CF₃ groups;
n is an integer of 0, 1 or 2;
Y is Cl or Br; and
A is hydrogen or C₁-C₄alkyl substituted with one C₁-C₄alkoxy group.

A third group of more preferred arylthio, -sulfinyl and -sulfonyl pyrrole compounds of the present invention are those having the structural formula IV 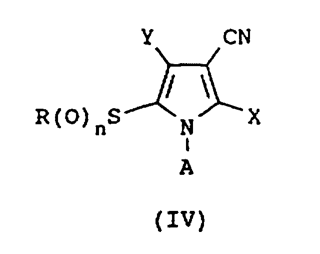 wherein
R and X are each independently phenyl optionally substituted with any combination of from one to three halogen, NO₂ or CF₃ groups;
n is an integer of 0, 1 or 2;
Y is hydrogen, Cl or Br; and
A is hydrogen or C₁-C₄alkyl substituted with one C₁-C₄alkoxy group.

Formula I compounds of the present invention which are particularly effective insecticidal agents include
2-(*p*-chlorophenyl)-4-[(3,4-dichlorophenyl)thio]-5-(trifluoromethyl)pyrrole-3-carbonitrile;
2-(*p*-chlorophenyl)-4-[(*p*-chlorophenyl)thio]-5-(trifluoromethyl)pyrrole-3-carbonitrile;
3-bromo-4-[(3-chloro-4-fluorophenyl)sulfonyl]-5-(*p*-chlorophenyl)-2-(trifluoromethyl)pyrrole;
3-bromo-5-(*p*-chlorophenyl)-4-[(*p*-chlorophenyl)sulfonyl]-2-(trifluoromethyl)pyrrole;
4-bromo-2-(*p*-chlorophenyl)-5-[(*p*-chlorophenyl)sulfonyl]pyrrole-3-carbonitrile;
2-(*p*-chlorophenyl)-5-[(3,4-dichlorophenyl)sulfonyl]-pyrrole-3-carbonitrile; and
2-(*p*-chlorophenyl)-5-[(3,4-dichlorophenyl)sulfonyl]-1-(ethoxymethyl)pyrrole-3-carbonitrile, among others.

Exemplary of halogen hereinabove are fluorine, chlorine, bromine and iodine. The terms "C₁-C₄haloalkyl" and "C₁-C₄haloalkoxy" are defined as a C₁-C₄alkyl group and a C₁-C₄alkoxy group substituted with one or more halogen atoms, respectively.

Advantageously, it has been found that the formula I compounds of the present invention are especially useful for the control of tobacco budworms and southern armyworms.

Formula I compounds wherein A is hydrogen and R, W, X, Y and n are as described hereinabove for formula I may be prepared by reacting a substituted pyrrole of formula V with an arylsulfenyl halide of formula VI optionally in the presence of a base to form an arylthio pyrrole of formula Ia, and optionally oxidizing the arylthio pyrrole with a suitable oxidizing agent such as hydrogen peroxide, 3-chloroperoxybenzoic acid, potassium peroxymonosulfate and the like to obtain an arylsulfinyl pyrrole of formula Ib or an arylsulfonyl pyrrole of formula Ic. The reaction scheme is shown below in Flow Diagram I.

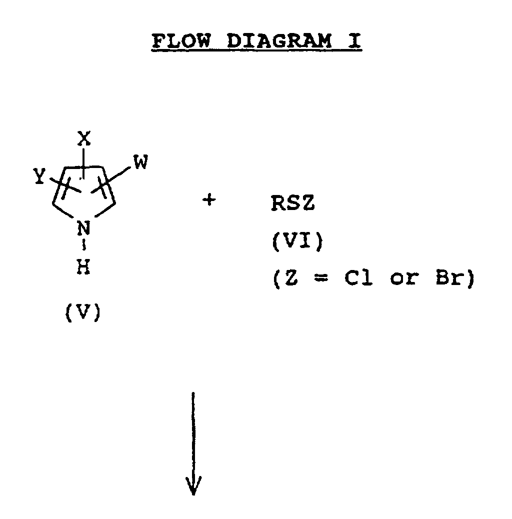

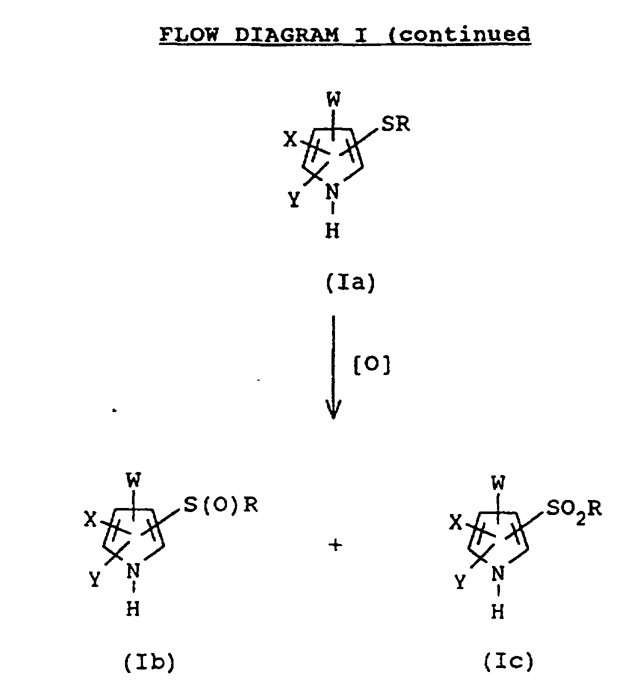

1-Substituted formula I compounds may be prepared by reacting a formula I compound wherein A is hydrogen with an alkylating or acylating agent in the presence of an alkali metal alkoxide or hydride. For example, a formula I compound wherein A is hydrogen and R, W, X, Y and n are as described for formula I above is reacted with an alkylating agent such as a C₁-C₆alkylhalide in which the alkyl group is straight or branched and is optionally substituted with one to three halogen atoms, one hydroxy, one cyano, one C₁-C₄alkoxy, one C₁-C₄alkylthio, one phenyl optionally substituted with one to three halogen atoms, or one benzyloxy group optionally substituted with one to three halogen atoms, and an alkali metal alkoxide such as sodium or potassium tert-butoxide. That reaction provides an arylthio, -sulfinyl or -sulfonyl pyrrole having the same substituents as the starting material, but in addition is substituted on the nitrogen atom with a C₁-C₆alkyl group optionally substituted as described above. The reaction scheme is shown in Flow Diagram II. 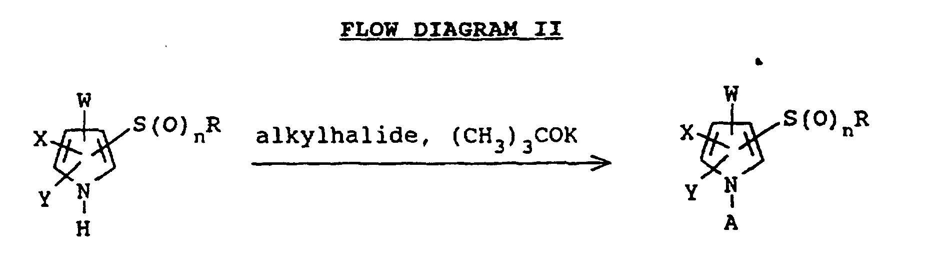 wherein R, W, X, Y and n are as described for formula I above and A is C₁-C₆alkyl optionally substituted as described above. In a similar reaction, cyanogen bromide is substituted for the alkylhalide and provides a formula I arylthio, -sulfinyl or -sulfonyl pyrrole wherein A is cyano.

Advantageously, the above-described alkylation procedure may also be applied for the preparation of formula I compounds wherein A is C₃-C₆alkenyl or C₃-C₆alkynyl. This is achieved by substituting a C₃-C₆alkenyl halide or C₃-C₆alkynyl halide for the C₁-C₆alkyl halide used in the above-described reaction.

In a similar manner, 1-acylated arylthio, -sulfinyl and -sulfonyl pyrroles may be prepared by reacting a formula I compound wherein A is hydrogen with an acylating agent in the presence of an alkali metal alkoxide. Acylating agents such as optionally substituted C₁-C₆alkyl and C₂-C₆alkenyl acid chlorides, an optionally substituted benzoyl chloride, an optionally substituted phenylchloroformate, optionally substituted C₁-C₆alkyl- and C₂-C₆alkenylchloroformates, an N-substituted carbamoyl chloride and the like may be used. The reaction is shown in Flow Diagram III.

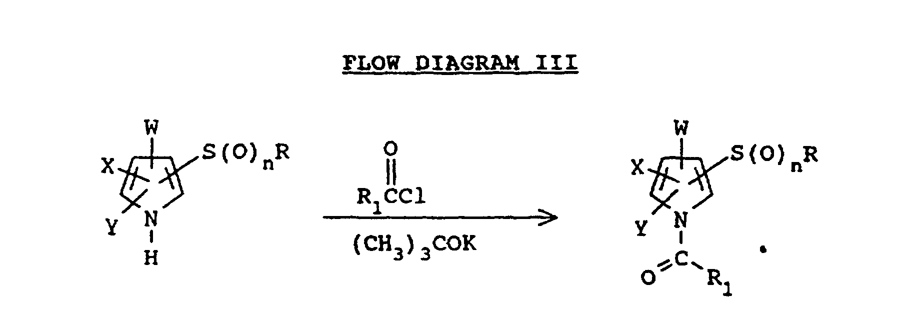

Formula I compounds wherein A is CH₂SQ may be prepared by reacting a formula I compound wherein A is chloromethyl with an alkali metal salt of an SQ compound in the presence of a base. And formula I compounds wherein A is CHR₂NHC(O)R₃ may be prepared as shown below in Flow Diagram IV.

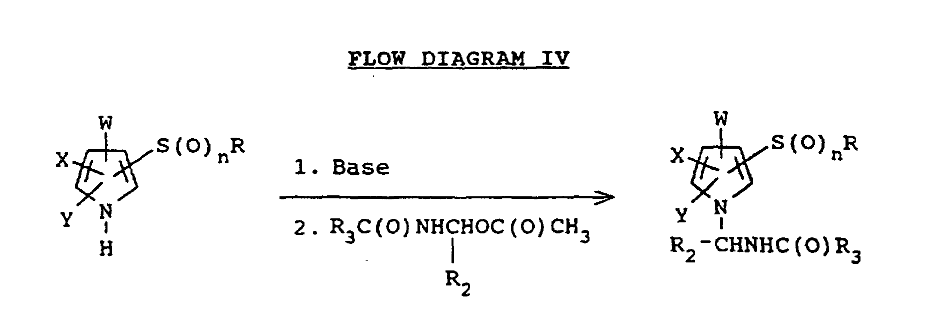

Advantageously, 1-halomethyl pyrroles of this invention may be prepared by reacting a formula I compound wherein A is CH(R₂)NHC(O)R₃ with a phosphorus oxyhalide compound such as phosphorus oxychloride. The reaction scheme is shown in Flow Diagram V.

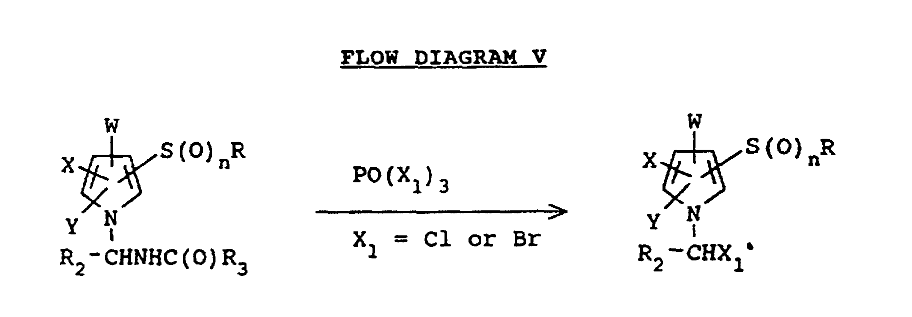

Formula I compounds wherein A is CHR₄OC(O)(CR₅R₆)ₘQ₁ may be prepared by reacting a 1-halomethyl pyrrole of this invention with a Q₁(CR₅R₆)ₘCO₂H compound in the presence of a base such as sodium hydroxide. The reaction is shown in Flow Diagram VI.

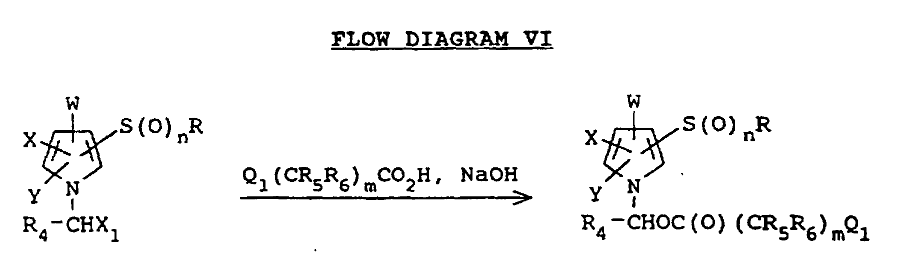

Starting formula V pyrrole compounds are known in the art (see, e.g., U.S. 5,010,098; U.S. 5,281,719 and CA-A1-2,076,937).

The arylthio, -sulfinyl and -sulfonyl pyrrole compounds of the present invention are effective for controlling undesirable insect species. Those compounds are also effective for protecting growing or harvested crops from attack by insects.

Insects which may be controlled by the formula I compounds of this invention include Lepidoptera such as tobacco budworms, cabbage loopers, cotton boll worms, beet armyworms, southern armyworms and diamondback moths; Homoptera such as aphids, leaf hoppers, plant hoppers and white flies; Thysanoptera such as thrips; Coleoptera such as boll weevils, Colorado potato beetles, southern corn rootworms and mustard beetles; and Orthoptera such as locusts, crickets, grasshoppers and cockroaches. The compounds of the present invention are especially effective against Lepidoptera such as tobacco budworms and southern armyworms.

Surprisingly, it has been found that, in general, the compounds of the present invention are not effective for the control of mites. That property makes the compounds of the present invention especially suitable for use in integrated pest management systems which utilize predatory mites.

In practice generally about 10 ppm to about 10,000 ppm and preferably about 100 ppm to about 5,000 ppm of a formula I arylthio, -sulfinyl or -sulfonyl pyrrole, dispersed in water or another liquid carrier, is effective when applied to the plants, the crops or the soil in which said crops are growing to protect said crops from attack by insects.

The arylthio, -sulfinyl and -sulfonyl pyrrole compounds of this invention are also effective for controlling insects when applied to the foliage of plants and/or to the soil or water in which said plants are growing in sufficient amount to provide a rate of about 0.1 kg/ha to 4.0 kg/ha of active ingredient.

While the compounds of this invention are effective for controlling insects when employed alone, they may also be used in combination with other biological chemicals, including other insecticides. For example, the formula I compounds of this invention may be used effectively in conjunction or combination with pyrethroids, phosphates, carbamates, cyclodienes, endotoxin of bacillus thuringiensis (Bt), formamidines, phenol tin compounds, chlorinated hydrocarbons, benzoylphenyl ureas and the like.

The compounds of this invention may be formulated as emulsifiable concentrates, flowable concentrates or wettable powders which are diluted with water or other suitable polar solvent, generally *in situ*, and then applied as a dilute spray. Said compounds may also be formulated in dry compacted granules, granular formulations, dusts, dust concentrates, suspension concentrates, microemulsions and the like all of which lend themselves to seed, soil, water and/or foliage applications to provide the requisite plant protection. Such formulations include the compounds of the invention admixed with agronomically acceptable inert, solid or liquid carriers.

In order to facilitate a further understanding of the invention, the following examples are presented primarily for the purpose of illustrating more specific details thereof. The examples generally utilize the above reaction schemes and also provide further means for preparing compounds of the present invention.

### EXAMPLE 1

### Preparation of 2-(p-Chlorophenyl)-3-[(p-chlorophenyl)thio]-5-(trifluoromethyl)pyrrole

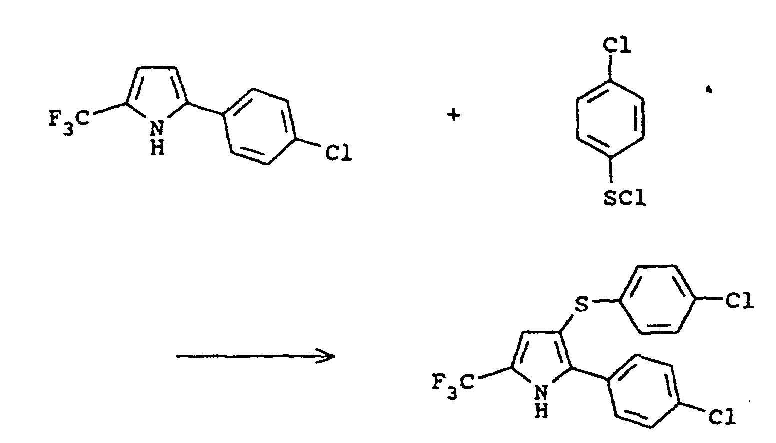

A solution of *p*-chlorophenylsulfenyl chloride (0.80 g, 4.48 mmol) in methylene chloride is added dropwise over 20 minutes to a stirred solution of 2-(*p*-chlorophenyl)-5-trifluoromethylpyrrole (1.0 g, 4.07 mmol) in methylene chloride. The reaction mixture is stirred at room temperature for 90 minutes, diluted with methylene chloride, washed sequentially with water and brine, dried over anhydrous magnesium sulfate and concentrated *in vacuo* to obtain a residue. Flash column chromatography of the residue using silica gel and a 1:10 ethyl acetate/ hexanes solution gives the title product as an off-white solid (0.96 g, mp 39-54 °C).

Using essentially the same procedure, the following compounds are obtained:

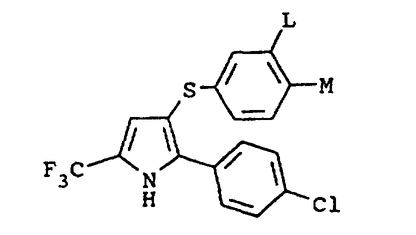

| **L** | **M** | **State/mp °C** |
|---|---|---|
| H | H | syrup |
| Cl | F | syrup |
| H | NO₂ | 112 |

### EXAMPLE 2

### Preparation of 2-(p-Chlorophenyl)-3-[(p-chlorophenyl)sulfonyl]-5-(trifluoromethyl)pyrrole

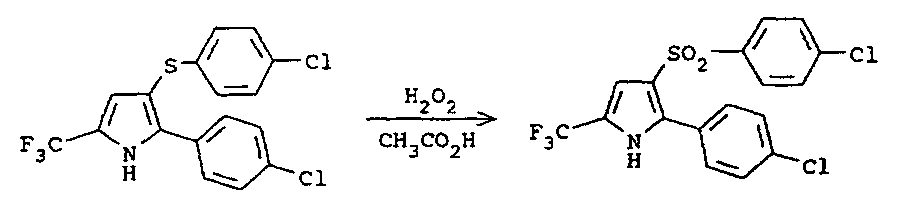

A solution of 2-(*p*-chlorophenyl)-3-[(*p*-chlorophenyl)thio]-5-(trifluoromethyl)pyrrole (1.0 g, 2.58 mmol) and 30% hydrogen peroxide solution (0.88 g, 7.73 mmol) in acetic acid is stirred at room temperature for 1 hour, stirred at 75 °C for 2 hours, cooled to room temperature and poured into water. The resultant aqueous mixture is filtered to obtain the title product as a white solid (0.62 g, mp 200-206 °C).

Using essentially the same procedure, the following compounds are obtained:

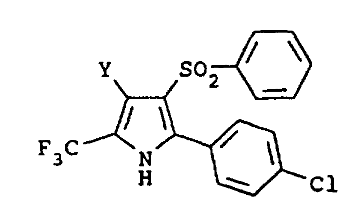

| **Y** | **mp °C** |
|---|---|
| H | 153-156 |
| Br | 167-169 |

### EXAMPLE 3

### Preparation of 3-Bromo-5-(p-chlorophenyl)-4-[(p-chlorophenyl)sulfonvll-2-(trifluoromethyl)pyrrole

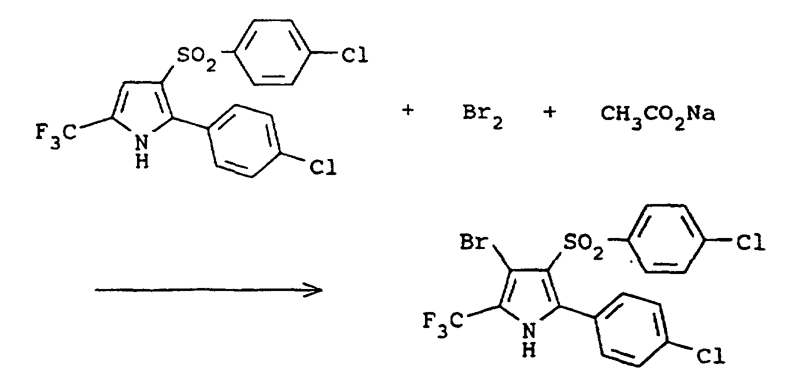

A solution of 2-(*p*-chlorophenyl)-3-[(*p*-chlorophenyl)sulfonyl]-5-(trifluoromethyl)pyrrole (2.15 g, 5.12 mmol), bromine (0.29 mL, 5.63 mmol) and sodium acetate (0.46 g, 5.63 mmol) in acetic acid is stirred at room temperature for 4 hours, treated with additional bromine (3 drops), stirred overnight at room temperature and poured into water. The resultant aqueous mixture is filtered to obtain a solid. Flash column chromatography of the solid using silica gel and a 1:5 ethyl acetate/ hexanes solution gives the title product as a white solid (1.88 g, mp >230 °C).

Using essentially the same procedure, the following compounds are obtained:

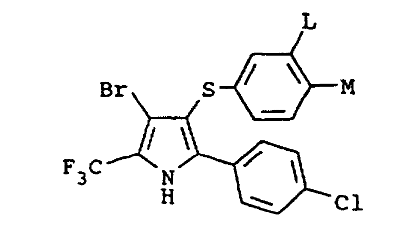

| **L** | **M** | **State/mp °C** |
|---|---|---|
| H | CL | syrup |
| H | H | syrup |
| Cl | F | 181-184 |

### EXAMPLE 4

### Preparation of 3-Bromo-5-(p-chlorophenyl)-4-[(p-chlorophenyl)sulfonyl]-1-(ethoxymethyl)-2-(trifluoromethyl)pyrrole

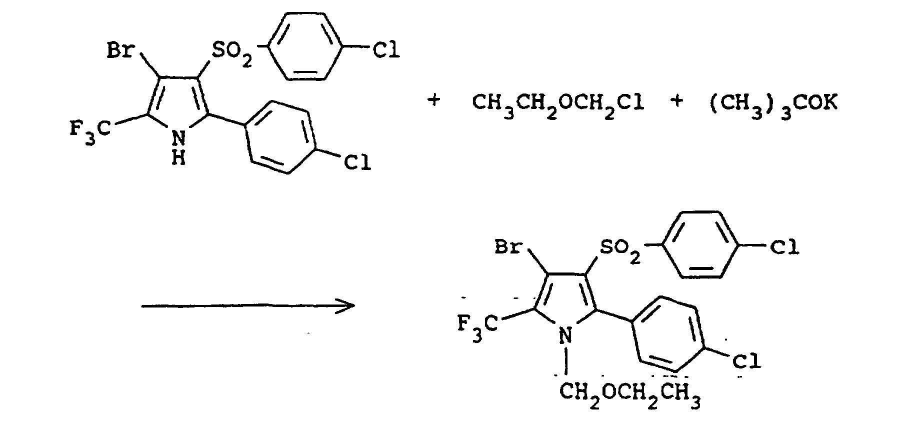

A solution of 3-bromo-5-(*p*-chlorophenyl)-4-[(*p*-chlorophenyl)sulfonyl]-2-(trifluoromethyl)pyrrole (0.92 g, 1.84 mmol) and potassium *tert*-butoxide (0.26 g, 2.21 mmol) in tetrahydrofuran is stirred at room temperature for 5 minutes, treated with a solution of chloromethyl ethyl ether (0.21 g, 2.21 mmol) in tetrahydrofuran, stirred at room temperature overnight, diluted with ethyl acetate, washed sequentially with water and brine, dried over anhydrous magnesium sulfate and concentrated in *vacuo* to give the title product as a white solid (0.98 g, mp 107-112 °C).

Using essentially the same procedure, the following compounds are obtained:

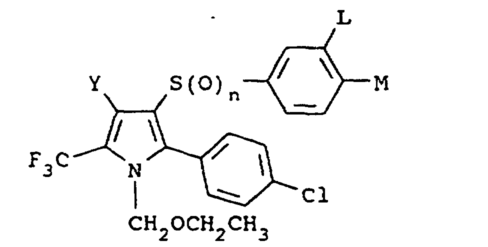

| **Y** | **L** | **M** | **n** | **State/ mp °C** |
|---|---|---|---|---|
| H | H | Cl | 1 | 95-100 |
| H | H | Cl | 2 | 108-110 |
| Br | H | Cl | 1 | syrup |
| H | H | H | 0 | syrup |
| H | H | H | 1 | syrup |
| Br | H | H | 0 | 96-98 |
| H | H | H | 2 | syrup |
| Cl | H | H | 0 | syrup |
| H | Cl | F | 0 | 56-58 |
| H | Cl | F | 1 | syrup |
| Br | Cl | F | 1 | 111-114 |
| H | H | NO₂ | 0 | 76-77 |
| Br | Cl | F | 0 | 80-83 |

### EXAMPLE 5

### Preparation of 2-(p-Chlorophenyl)-3-[(p-chlorophenyl)sulfinyl]-5-(trifluoromethyl)pyrrole

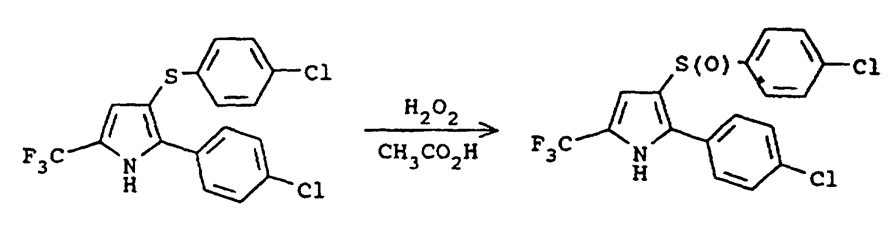

A solution of 2-(*p*-chlorophenyl)-3-[(*p*-chlorophenyl)thio]-5-(trifluoromethyl)pyrrole (2.57 g, 6.6 mmol) and 30% hydrogen peroxide solution (0.83 g, 7.3 mmol) in acetic acid is stirred at room temperature overnight and poured into water. The resultant aqueous mixture is filtered to obtain a solid. A solution of the solid in methylene chloride is washed with brine, dried over anhydrous magnesium sulfate and concentrated *in vacuo* to obtain a residue. Flash column chromatography of the residue using silica gel and a 1:3 ethyl acetate/hexanes solution gives the title product as a white solid (1.75 g, mp 174-176 °C).

Using essentially the same procedure, the following compounds are obtained:

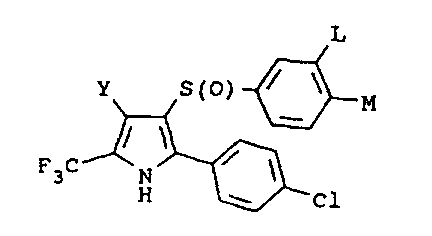

| **Y** | **L** | **M** | **mp °C** |
|---|---|---|---|
| H | H | H | 141-144 |
| Br | H | H | 96-98 |
| Br | H | Cl | 162-164 |
| Cl | H | H | 151-153 |
| H | Cl | F | 180-183 |
| Br | Cl | F | 169-172 |

### EXAMPLE 6

### Preparation of 3-Chloro-5-(p-chlorophenyl)-4-(phenylthio)-2-(trifluoromethyl)pyrrole

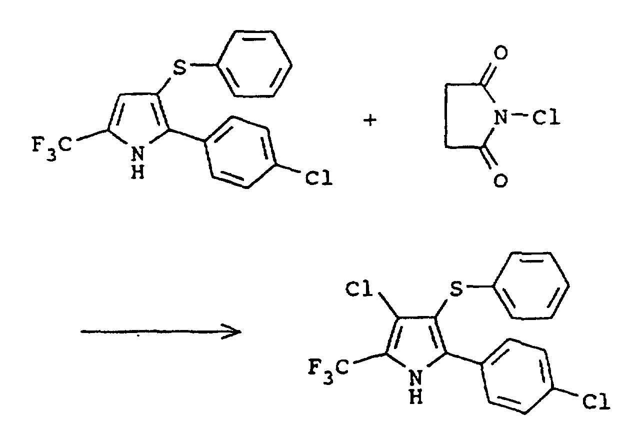

N-Chlorosuccinimide (0.734 g, 5.5 mmol) is added to a solution of 2-(*p*-chlorophenyl)-3-(phenylthio)-5-(trifluoromethyl)pyrrole (1.77 g, 5 mmol) in tetrahydrofuran. The reaction mixture is stirred overnight at room temperature, concentrated *in vacuo,* diluted with carbon tetrachloride and filtered. The resultant filtrate is concentrated *in vacuo* to obtain a residue. Flash column chromatography of the residue using silica gel and a 1:10 ethyl acetate/hexanes solution gives the title product as a yellow syrup which is identified by ¹H NMR spectral analysis.

### EXAMPLE 7

### Preparation of 2-(p-Chlorophenyl)-5-[(p-nitrophenyl)thio]pyrrole-3-carbonitrile

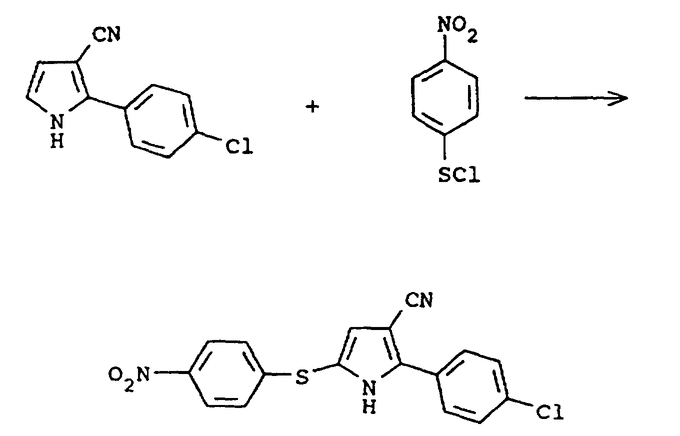

A mixture of 2-(*p*-chlorophenyl)pyrrole-3-carbonitrile (3.01 g, 1.5 mmol) in methylene chloride is cooled to 0 °C, treated dropwise over 30 minutes with a solution of *p*-nitrophenylsulfenyl chloride (2.98 g, 1.57 mmol) in methylene chloride, stirred at room temperature for 90 minutes and concentrated *in vacuo* to obtain a residue. A solution of the residue in ethyl acetate is washed sequentially with water and brine, dried over anhydrous sodium sulfate and concentrated *in vacuo* to obtain a solid. The solid is recrystallized from methanol to give the title product as a yellow solid (4.11 g, mp >230 °C).

Using essentially the same procedure, the following compounds are obtained:

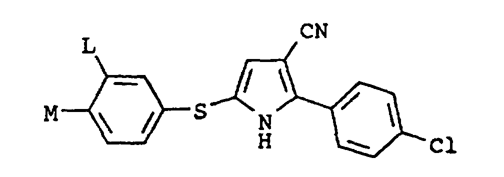

| **L** | **M** | **mp °C** |
|---|---|---|
| H | Cl | 190-193 |
| H | H | 165-168 |
| Cl | Cl | 209-211 |
| Cl | F | 161-163 |

### EXAMPLE 8

### Preparation of 2-(p-Chlorophenyl)-5-[(p-chlorophenyl)thio]-3-nitropyrrole

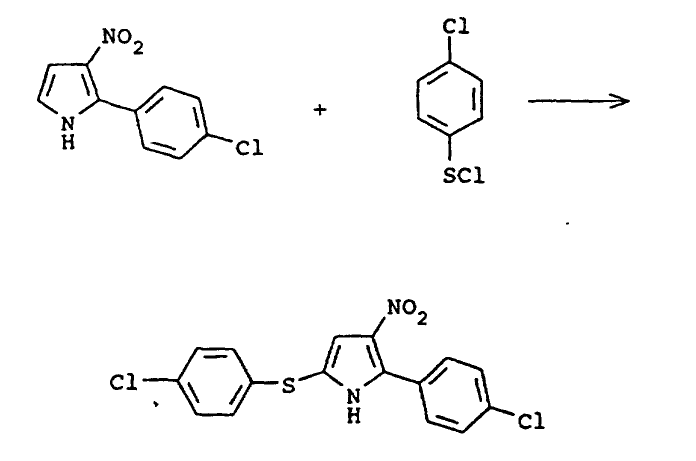

A solution of 2-(*p*-chlorophenyl)-3-nitropyrrole (4.88 g, 21.9 mmol) in methylene chloride is cooled to 0 °C, treated dropwise over 30 minutes with a solution of *p*-chlorophenylsulfenyl chloride (4.32 g, 24 mmol) in methylene chloride, stirred at 0 °C for 3 hours, stirred overnight at room temperature, diluted with methylene chloride, washed sequentially with water, saturated sodium bicarbonate solution and brine, dried over anhydrous magnesium sulfate and concentrated in *vacuo* to obtain a dark residue. Flash column chromatography of the residue using silica gel and a 1:5 ethyl acetate/hexanes solution gives the title product as a yellow solid (2.38 g, mp 195-197 °C).

Using essentially the same procedure, but substituting 3,4-dichlorophenylsulfenyl chloride for *p*-chlorophenylsulfenyl chloride, 2-(*p*-chlorophenyl)-5-[(3,4-dichlorophenyl)thio]-3-nitropyrrole is obtained as a yellow solid.

### EXAMPLE 9

### Preparation of 2-(p-Chlorophenyl)-5-(phenylsulfinyl)pyrrole-3-carbonitrile

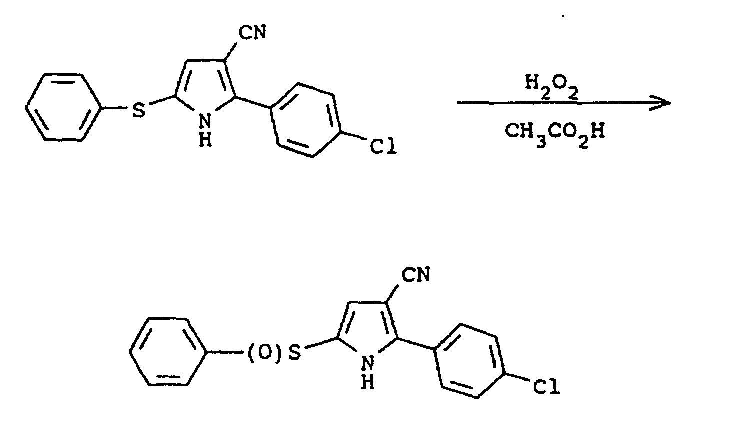

A mixture of 2-(*p*-chlorophenyl)-5-(phenylthio)-pyrrole-3-carbonitrile (916 mg, 2.95 mmol) and 30% hydrogen peroxide solution (105.3 mg, 3.09 mmol) in acetic acid is stirred at room temperature for 3 days, diluted with water and filtered to obtain a solid. The solid is recrystallized from methanol to give the title product as a grey solid (510 mg, mp 169-170 °C).

Using essentially the same procedure, the following compounds are obtained:

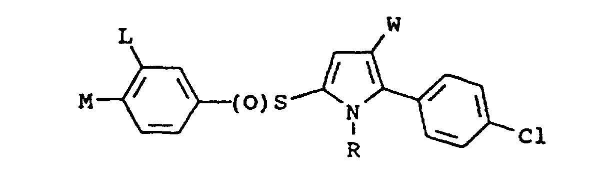

| **L** | **M** | **W** | **R** | **State/ mp °C** |
|---|---|---|---|---|
| H | H | NO₂ | H | 187-190 |
| Cl | Cl | CN | CH₂OC₂H₅ | 90-92 |
| Cl | F | CN | CH₂OC₂H₅ | syrup |

### EXAMPLE 10

### Preparation of 2-(p-Chlorophenyl)-5-[(3,4-dichlorophenyl)thio]-1-(ethoxymethyl)pyrrole-3-carbonitrile

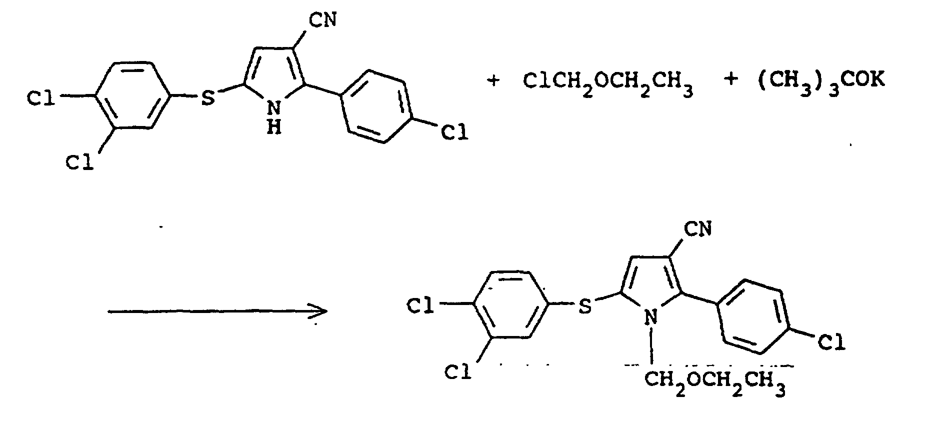

A mixture of 2-(*p*-chlorophenyl)-5-[(3,4-dichlorophenyl)thio]pyrrole-3-carbonitrile (820 mg, 2.16 mmol) and potassium *tert*-butoxide (266 mg, 2.37 mmol) in tetrahydrofuran is stirred at room temperature for 30 minutes, treated with chloromethyl ethyl ether (225 mg, 2.37 mmol), stirred at room temperature for 4 hours, and diluted with water and ethyl acetate. The phases are separated and the aqueous phase is extracted with ethyl acetate. The organic phase and extracts are combined, washed with water, dried over anhydrous sodium sulfate and concentrated *in vacuo* to obtain a syrup. The syrup is crystallized from an ethyl acetate/hexanes solution to give the title product as a solid (780 mg, mp 95-97 °C).

Using essentially the same procedure, the following compounds are obtained:

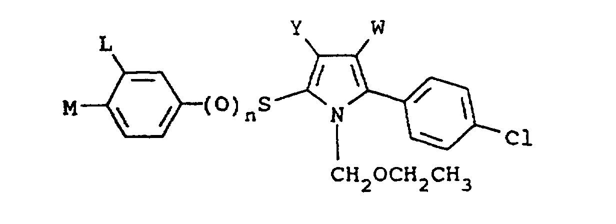

| **W** | **Y** | **L** | **M** | **n** | **State/ mp °C** |
|---|---|---|---|---|---|
| CN | H | H | Cl | 0 | 124-125 |
| CN | Br | H | Cl | 0 | 117.5-119.5 |
| CN | H | H | Cl | 2 | 107-109.5 |
| CN | H | H | NO₂ | 0 | 122-124 |
| CN | H | H | NO₂ | 2 | 187-190 |
| CN | Br | H | NO₂ | 0 | 142-145 |
| CN | H | H | H | 0 | syrup |
| CN | H | Cl | Cl | 2 | 131-133 |
| CN | H | Cl | F | 0 | 86-88.5 |
| CN | H | Cl | F | 2 | 128-130 |
| CN | Br | Cl | F | 2 | 156-159 |
| NO₂ | H | H | Cl | 2 | |

### EXAMPLE 11

### Preparation of 2-(p-Chlorophenyl)-5-[(p-chlorophenyl)sulfonyl]pyrrole-3-carbonitrile

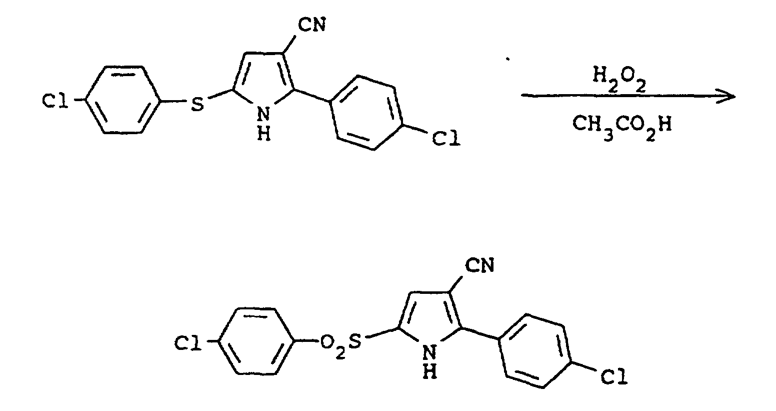

A mixture of 2-(*p*-chlorophenyl)-5-[(*p*-chlorophenyl)thio]pyrrole-3-carbonitrile (1.035 g, 3 mmol) and 30% hydrogen peroxide solution (0.102 g, 9 mmol) in acetic acid is stirred at room temperature overnight, stirred at 55 °C for 5 hours and poured into an ice-water mixture. The resultant aqueous mixture is filtered to obtain a solid which is washed with water and dried overnight in a vacuum oven at 60 °C to give the title product as a colorless solid (1.03 g, mp 159-160 °C).

Using essentially the same procedure, the following compounds are obtained:

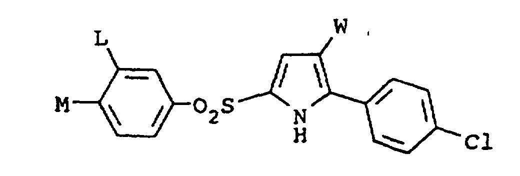

| **L** | **M** | **W** | **mp °C** |
|---|---|---|---|
| H | Cl | NO₂ | 228-230 |
| H | NO₂ | CN | 178 |
| Cl | Cl | CN | >230 |
| Cl | F | CN | 232-235 |

### EXAMPLE 12

### Preparation of 4-Bromo-2-(p-chlorophenyl)-[5-(p-chlorophenyl)thio]pyrrole-3-carbonitrile

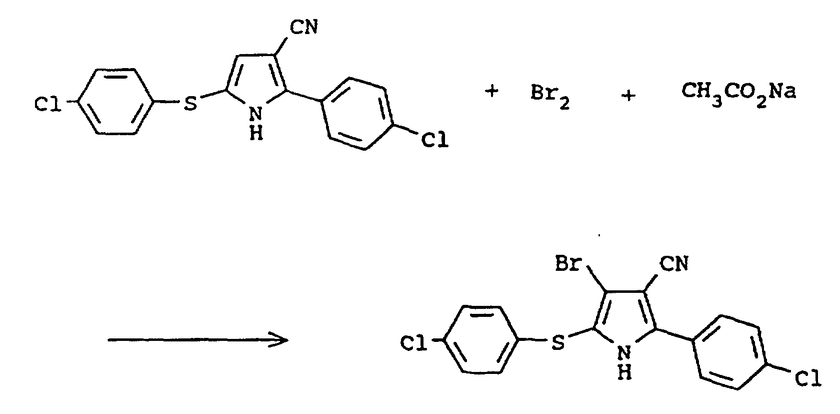

A mixture of 2-(*p*-chlorophenyl)-5-[(*p*-chlorophenyl)thio]pyrrole-3-carbonitrile (0.831 g, 2.4 mmol), bromine (0.423 g, 2.7 mmol) and sodium acetate (0.217 g, 2.7 mmol) in acetic acid is stirred at room temperature for 1 hour and poured into water. The resultant aqueous mixture is filtered to obtain a solid which is washed with water and recrystallized from 2-propanol to give the title product as colorless crystals (0.51 g, mp >230 °C).

Using essentially the same procedure, the following compounds are obtained:

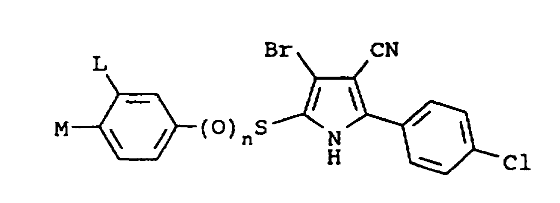

| **L** | **M** | **n** | **mp °C** |
|---|---|---|---|
| H | Cl | 2 | 159-160 |
| H | NO₂ | 2 | >230 |
| Cl | Cl | 0 | >230 |
| Cl | F | 2 | >230 |

### EXAMPLE 13

### Preparation of 4-Bromo-2-(p-chlorophenyl)-5-[(3,4-dichlorophenyl)thio]-1-(ethoxymethyl)pyrrole-3-carbonitrile

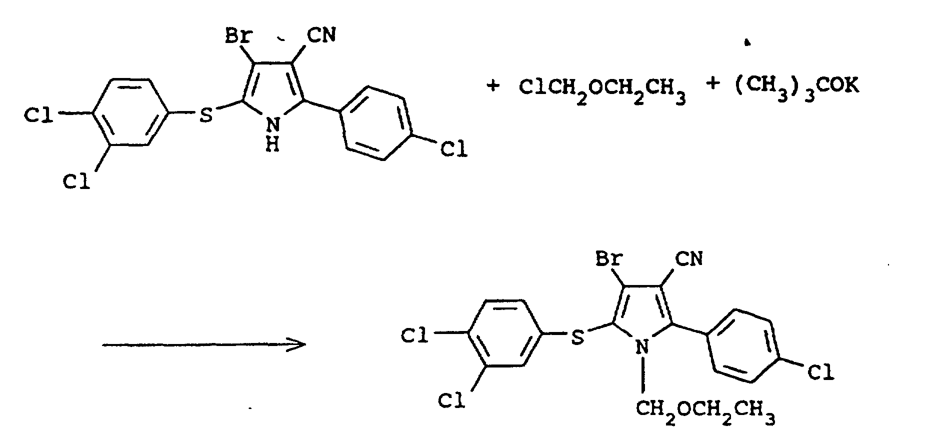

A solution of 4-bromo-2-(*p*-chlorophenyl)-5-[(3,4-dichlorophenyl)thio]pyrrole-3-carbonitrile (0.644 g, 1.40 mmol) and potassium *tert*-butoxide (0.173 g, 1.54 mmol) in tetrahydrofuran is stirred at room temperature for 30 minutes, treated with chloromethyl ethyl ether (0.16 mL, 1.54 mmol), stirred overnight at room temperature, diluted with ethyl acetate, washed with water, dried over anhydrous sodium sulfate and concentrated in vacuo to obtain a solid. The solid is recrystallized from an ethyl acetate/hexanes solution to give the title product as a white solid (0.554 g, mp 105-107 °C).

### EXAMPLE 14

### Preparation of 2-(p-Chlorophenyl)-4-[(3,4-dichlorophenyl)thio]-5-(trifluoromethyl)pyrrole-3-carbonitrile

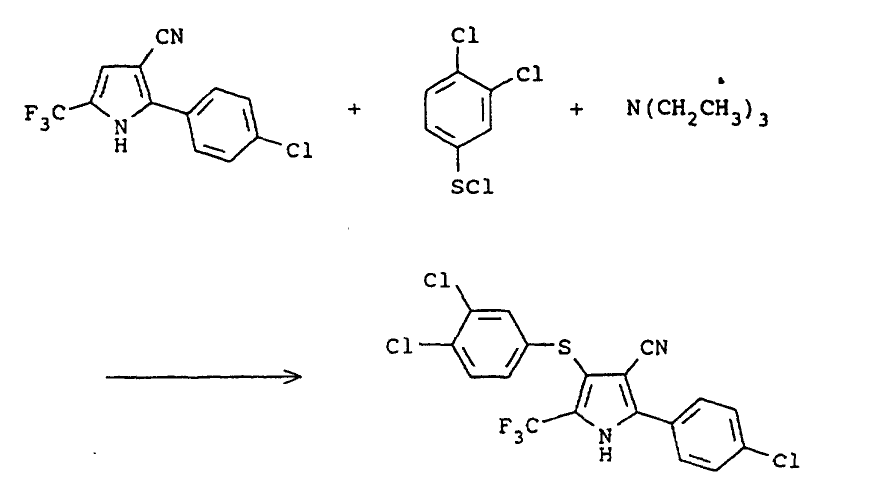

3,4-Dichlorophenylsulfenyl chloride (1.6 g, 7.49 mmol) is added to a solution of 2-(p-chlorophenyl)-5-trifluoromethylpyrrole-3-carbonitrile (2.02 g, 7.48 mmol) and triethylamine (1.67 g, 16.5 mmol) in methylene chloride. The reaction mixture is stirred at room temperature for 24 hours and concentrated *in vacuo* to obtain a residue. The residue is slurried in ethyl acetate and the resultant mixture is filtered to remove solids. The filtrate is diluted with hexanes to precipitate a solid which is collected and dried to give the title product as a cream colored solid (mp 94-97 °C).

Using essentially the same procedure, the following compounds are obtained:

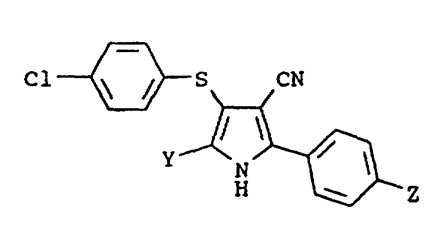

| **Y** | **Z** | **mp °C** |
|---|---|---|
| H | CF₃ | 155-156 |
| CF₃ | Cl | 214-217 |

### EXAMPLE 15

### Preparation of 2-(p-Chlorophenyl)-4-[(p-chlorophenyl)thio]-1-(ethoxymethyl)-5-(trifluoromethyl)pyrrole-3-carbonitrile

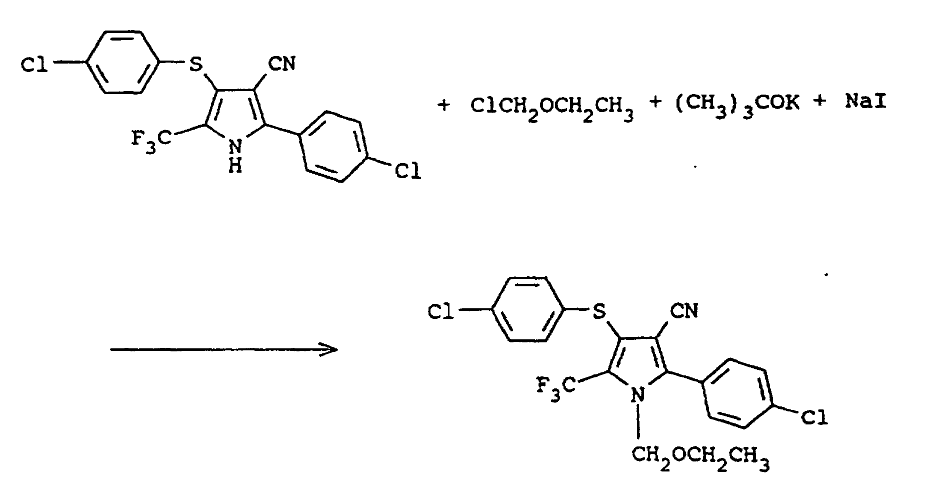

A mixture of 2-(*p*-chlorophenyl)-4-[(*p*-chlorophenyl)thio]-5-(trifluoromethyl)pyrrole-3-carbonitrile (1.7 g, 4.1 mmol), potassium *tert*-butoxide (0.5 g, 4.5 mmol) and sodium iodide (0.67 g, 4.5 mmol) in tetrahydrofuran is stirred at room temperature for 15 minutes, treated with chloromethyl ethyl ether (0.425 g, 4.5 mmol), stirred at room temperature for 6 hours and poured into water. The aqueous mixture is extracted with diethyl ether. The organic extract is washed sequentially with a 2 molar potassium hydroxide solution and water, dried over anhydrous sodium sulfate and concentrated *in vacuo* to obtain an oil. A mixture of the oil in hexanes is stored overnight at room temperature and filtered to obtain the title product as a white solid (1.25 g, mp 98-100 °C).

Using essentially the same procedure, 2-(*p*-chlorophenyl)-4-[(3,4-dichlorophenyl)thio]-1-(ethoxymethyl)-5-(trifluoromethyl)pyrrole-3-carbonitrile is obtained as a white solid, mp 75-77 °C.

### EXAMPLE 16

### Preparation of 2-(p-Chlorophenyl)-4-[(3,4-dichlorophenyl)sulfonyl]-5-(trifluoromethyl)pyrrole-3-carbonitrile

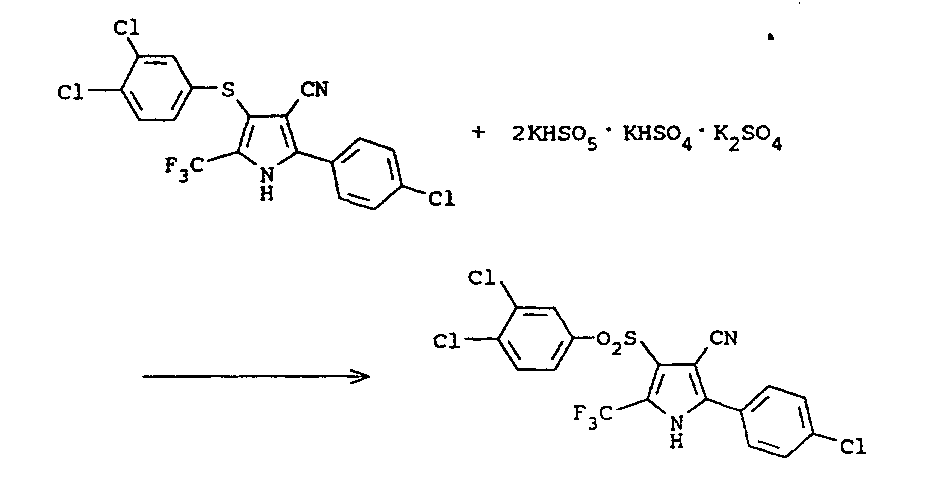

A solution of 2-(*p*-chlorophenyl)-4-[(3,4-dichlorophenyl)thio]-5-(trifluoromethyl)pyrrole-3-carbonitrile (1.0 g, 2.2 mmol) in methanol is treated with a solution of potassium peroxymonosulfate (2.95 g) in water (7.4 mL), stirred at 25 °C for 24 hours, diluted with additional methanol, treated with a solution of potassium peroxymonosulfate (2.7 g) in water (7.6 mL), stirred at 25 °C for 24 hours, and filtered to obtain a solid which is washed with water and dried to give the title product a white solid (mp >225 °C).

### EXAMPLE 17

### Preparation of 4'-Chloro-3-(1,3-dioxolan-2-yl)-2-(phenylthio)propiophenone

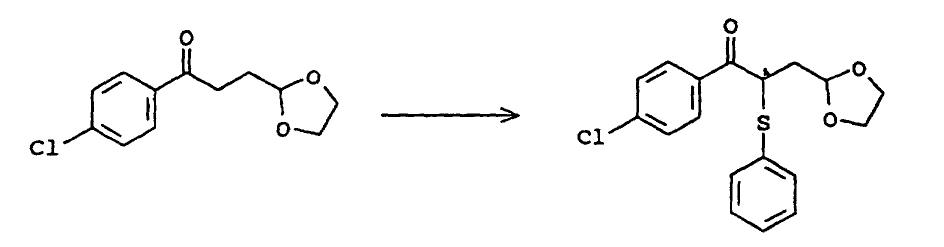

A solution of 4'-chloro-3-(1,3-dioxolan-2-yl)propiophenone (1.0 g, 4.16 mmol) in tetrahydrofuran is added dropwise to a solution of lithium diisopropylamide (3 mL of a 1.5 molar solution in tetrahydrofuran, 4.6 mmol) in tetrahydrofuran at -78 °C. The reaction mixture is stirred at -78 °C for 15 minutes, treated with a solution of phenyl disulfide (2.0 g, 9.2 mmol) in tetrahyrofuran, stirred at room temperature overnight, diluted with diethyl ether, washed sequentially with 1 N hydrochloric acid and brine, dried over anhydrous magnesium sulfate and concentrated *in vacuo* to obtain a residue. Flash column chromatography of the residue using silica gel and a 1:1 ethyl acetate/hexanes solution gives the title product as a white solid (1.0 g, mp 74-75 °C).

### EXAMPLE 18

### Preparation of 2-(p-Chlorophenyl)-3-(phenylthiolpyrrole

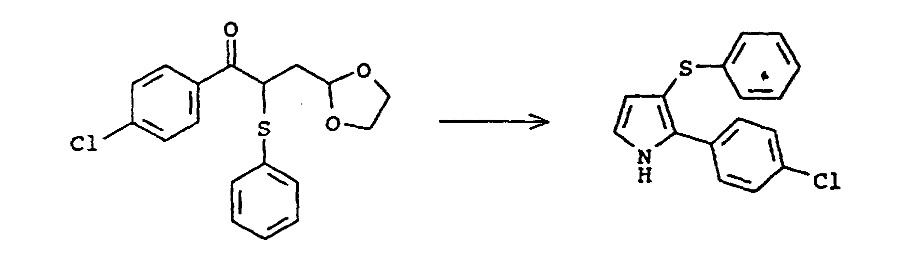

A solution of 4'-chloro-3-(1,3-dioxolan-2-yl)-2-(phenylthio)propiophenone (0.87 g, 2.5 mmol) and 4 N hydrochloric acid (15 mL) in acetone is stirred at 45 °C for 3 hours, cooled, and diluted with water and ethyl acetate. The organic phase is separated, washed with 5% sodium bicarbonate solution, dried over anhydrous sodium sulfate and concentrated *in vacuo* to obtain a residue. A solution of the residue in acetic acid is treated with ammonium acetate (3.0 g, 0.039 mol), stirred at 45 °C for 20 minutes, cooled to room temperature, and diluted with water and ethyl acetate. The organic phase is separated, dried over anhydrous sodium sulfate and concentrated *in vacuo* to obtain a dark residue. Flash column chromatography of the dark residue using silica gel and a 1:1 ethyl acetate/hexanes solution gives the title product as a dark brown syrup which is identified by NMR spectral analyses.

### EXAMPLE 19

### Preparation of 2-(p-Chlorophenyl)-3-(phenylsulfonyl)pyrrole

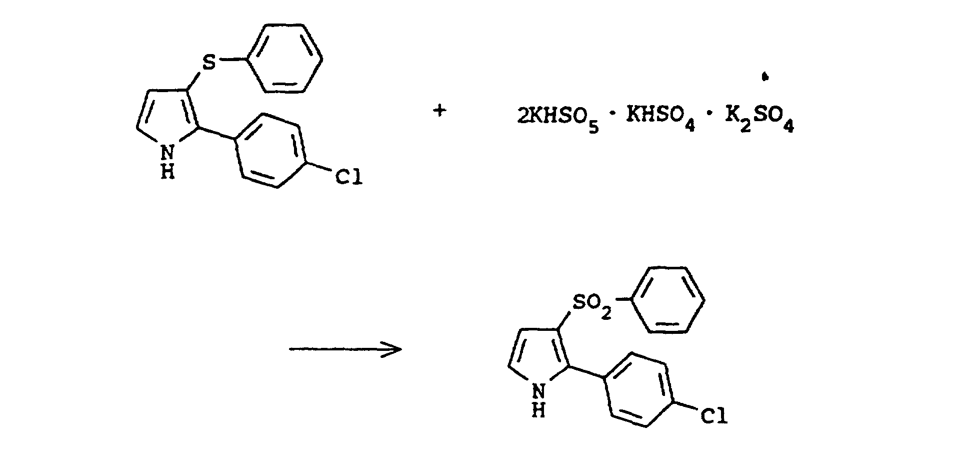

A solution of 2-(*p*-chlorophenyl)-3-(phenylthio)-pyrrole (1.0 g, 3.5 mmol) in methanol is treated with a solution of potassium peroxymonosulfate (2.95 g) in water (7.6 mL), stirred at room temperature for 5 hours, diluted with additional methanol, treated with a solution of potassium peroxymonosulfate (2.5 g) in water (7.4 mL), stirred at room temperature overnight and filtered to obtain a solid which is washed with water and dried to give the title product as a solid (mp 158-159 °C).

### EXAMPLE 20

### Preparation of 2,3-Dibromo-5-(p-chlorophenyl)-4-(phenylsulfonyl)pyrrole

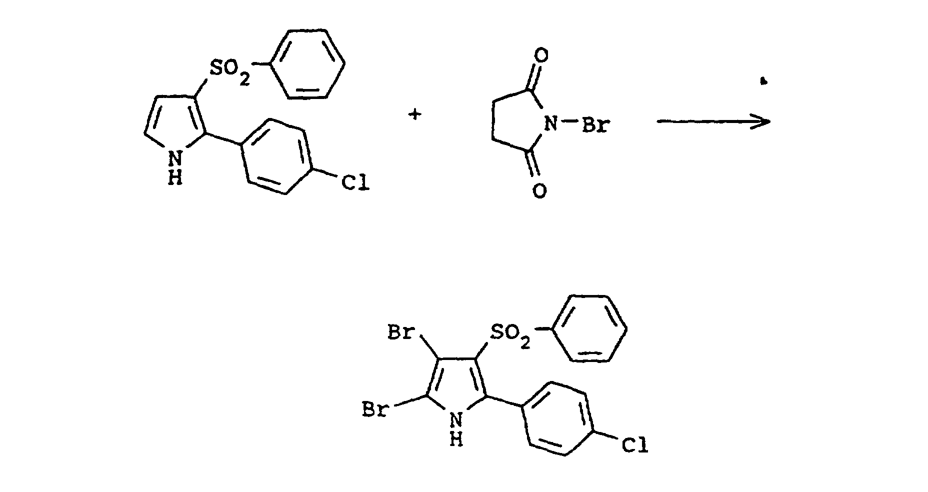

A mixture of 2-(*p*-chlorophenyl)-3-(phenylsulfonyl)-pyrrole (0.26 g, 0.82 mmol) and N-bromosuccinimide (0.35 g, 1.97 mmol) in tetrahydrofuran is stirred at room temperature for 30 hours and concentrated *in vacuo* to obtain a residue. Flash column chromatography of the residue using silica gel and a 1:1 ethyl acetate/hexanes solution gives the title product as a beige solid (0.24 g, mp >200 °C).

### EXAMPLE 21

### Preparation of 3-Chloro-5-(p-chlorophenyl)-4-(phenylsulfonyl)-2-(trifluoromethyl)pyrrole

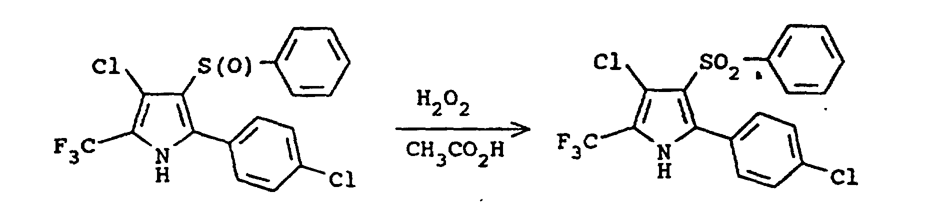

A mixture of 3-chloro-5-(*p*-chlorophenyl)-4-(phenylsulfinyl)-2-(trifluoromethyl)pyrrole (0.335 g, 0.83 mmol) and 30% hydrogen peroxide solution (0.28 mL, 2.49 mmol) in acetic acid is stirred at 50 °C for 7 hours, cooled and poured into an ice-water mixture. The resultant aqueous mixture is filtered to obtain a solid which is washed with water and dried to give the title product as a colorless solid (mp 158-161 °C).

Using essentially the same procedure, the following compounds are obtained:

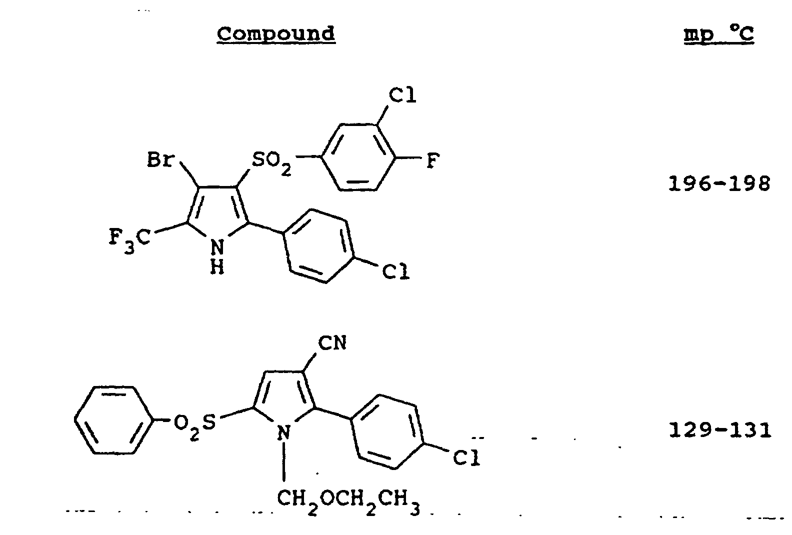

### EXAMPLE 22

### Insecticide Evaluations

The following tests show the efficacy of the com*pounds as* insecticides. The evaluations are conducted with solutions of test compounds dissolved or dispersed in 50:50 acetone/water mixtures. The test compound is technical material dissolved or dispersed in said acetone/water mixtures in sufficient amounts to provide the concentrations set forth in Table I below.

All concentrations reported herein are in terms of active ingredient. All tests are conducted in a laboratory maintained at about 27 °C. The rating system employed is as follows:

| **Rating System** | |
|---|---|
| 0 = no effect | 5 = 56-65% kill |
| 1 = 10-25% kill | 6 = 66-75% kill |
| 2 = 26-35% kill | 7 = 76-85% kill |
| 3 = 36-45% kill | 8 = 86-99% kill |
| 4 = 46-55% kill | 9 = 100% kill |
| | - = no evaluation |

The test species of insects used in the present evaluations along with specific test procedures are described below.

### Spodoptera eridania third instar larvae, southern armyworm

A sieva lima bean leaf expanded to 7 to 8 cm in length is dipped in the test suspension with agitation for three seconds and placed in a hood to dry. The leaf is then placed in a 100x10 mm petri dish containing a damp filter paper on the bottom and ten third instar caterpillars. The dish is maintained for five days before observations are made of mortality, reduced feeding or any interference with normal moulting.

### Heliothis virescens, third instar tobaaco budworm

Cotton cotyledons are dipped in the test formulation and allowed to dry in a hood. When dry, each is cut into quarters and ten sections placed individually in 30 mL plastic medicine cups containing a 5 to 7 mm long piece of damp dental wick. One third instar caterpillar is added to each cup and a cardboard lid placed on the cup. Treatments are maintained for three days before mortality counts and estimates of reduction in feeding damage are made.

Compounds employed in the above described insecticide evaluations are given a compound number and identified by name. Date in Table I are reported by compound number.

| **COMPOUNDS EVALUATED AS INSECTICIDAL AGENTS** | |
|---|---|
| **Compound Number** | |
| 1 | 2-(*p*-Chlorophenyl)-4-[(3,4-dichlorophehyl)thio]-5-(trifluoromethyl)pyrrole-3-carbonitrile |
| 2 | 2-(*p*-Chlorophenyl)-4-[(p-chlorophenyl)thio]-5-(trifluoromethyl)pyrrole-3-carbonitrile |
| 3 | 3-Bromo-4-[(3-chloro-4-fluorophenyl)sulfonyl]-5-(*p*-chlorophenyl)-2-(trifluoromethyl)pyrrole |
| 4 | 3-Bromo-5-(*p*-chlorophenyl)-4-[(*p*-chlorophenyl)sulfonyl]-2-(trifluoromethyl)pyrrole |
| 5 | 4-Bromo-2-(*p*-chlorophenyl)-5-[(*p*-chlorophenyl)sulfonyl]pyrrole-3-carbonitrile |
| 6 | 2-(*p*-Chlorophenyl)-5-[(3,4-dichlorophenyl)sulfonyl]pyrrole-3-carbonitrile |
| 7 | 2-(*p*-Chlorophenyl)-5-[(3,4-dichlorophenyl)sulfonyl]-1-(ethoxymethyl)pyrrole-3-carbonitrile |
| 8 | 2,3-Dibromo-5-(p-chlorophenyl)-4-(phenylsulfonyl)pyrrole |
| 9 | 4-[(*p*-Chlorophenyl)thio]-2-(α,α,α-trifluoro-*p*-tolyl)pyrrole-3-carbonitrile |
| 10 | 2-(*p*-Chlorophenyl)-4-[(*p*-chlorophenyl)thio]-1-(ethoxymethyl)-5-(trifluoromethyl)pyrrole-3-carbonitrile |
| 11 | 2-(*p*-Chlorophenyl)-4-[(3,4-dichlorophenyl)thio]-1-(ethoxymethyl)-5-(trifluoromethyl)pyrrole-3-carbonitrile |
| 12 | 2-(*p*-Chlorophenyl)-4-[(3,4-dichlorophenyl)sulfonyl]-5-(trifluoromethyl)pyrrole-3-carbonitrile |
| 13 | 2-(*p*-Chlorophenyl)-3-[(*p*-chlorophenyl)thio]-5-(trifluoromethyl)pyrrole |
| 14 | 2-(*p*-Chlorophenyl)-3-[(*p*-chlorophenyl)sulfonyl]-5-(trifluoromethyl)pyrrole |
| 15 | 2-(*p*-Chlorophenyl)-5-[(*p*-chlorophenyl)thio]pyrrole-3-carbonitrile |
| 16 | 4-Bromo-2-(*p*-chlorophenyl)-[5-(*p*-chlorophenyl)thio]pyrrole-3-carbonitrile |
| 17 | 4-Bromo-2-(*p*-chlorophenyl)-5-[(*p*-chlorophenyl)thio]-1-(ethoxymethyl)pyrrole-3-carbonitrile |
| 18 | 2-(*p*-Chlorophenyl)-5-[(*p*-chlorophenyl)thio]-1-(ethoxymethyl)pyrrole-3-carbonitrile |
| 19 | 2-(p-Chlorophenyl)-5-[(*p*-chlorophenyl)sulfonyl]-1-(ethoxymethyl)pyrrole-3-carbonitrile |
| 20 | 2-(*p*-Chlorophenyl)-5-[(*p*-chlorophenyl)sulfonyl]pyrrole-3-carbonitrile |
| 21 | 3-Bromo-5-(*p*-chlorophenyl)-4-[(*p*-chlorophenyl)sulfonyl]-1-(ethoxymethyl)-2-(trifluoromethyl)pyrrole |
| 22 | 2-(*p*-Chlorophenyl)-3-[(*p*-chlorophenyl)sulfinyl]-5-(trifluoromethyl)pyrrole |
| 23 | 2-(*p*-Chlorophenyl)-3-[(*p*-chlorophenyl)sulfinyl]-1-(ethoxymethyl)-5-(trifluoromethyl)pyrrole |
| 24 | 3-Bromo-5-(*p*-chlorophenyl)-4-[(*p*-chlorophenyl)thio]-2-(trifluoromethyl)pyrrole |
| 25 | 2-(*p*-Chlorophenyl)-5-[(*p*-chlorophenyl)thio]-3-nitropyrrole |
| 26 | 2-(*p*-Chlorophenyl)-5-[(*p*-chlorophenyl)sulfonyl]-1-(ethoxymethyl)-3-nitropyrrole |
| 27 | 2-(*p*-Chlorophenyl)-5-[(*p*-chlorophenyl)sulfonyl]-3-nitropyrrole |
| 28 | 2-(*p*-Chlorophenyl)-5-[(*p*-chlorophenyl)sulfinyl]-3-nitropyrrole |
| 29 | 2-(*p*-Chlorophenyl)-5-[(*p*-nitrophenyl)thio]pyrrole-3-carbonitrile |
| 30 | 2-(*p*-Chlorophenyl)-1-(ethoxymethyl)-5-[(*p*-nitrophenyl)thio]pyrrole-3-carbonitrile |
| 31 | 2-(*p*-Chlorophenyl)-5-[(*p*-nitrophenyl)sulfonyl)pyrrole-3-carbonitrile |
| 32 | 2-(*p*-Chlorophenyl)-1-(ethoxymethyl)-5-[(*p*-nitrophenyl)sulfonyl]pyrrole-3-carbonitrile |
| 33 | 4-Bromo-2-(*p*-chlorophenyl)-5-[(*p*-nitrophenyl)sulfonyl]pyrrole-3-carbonitrile |
| 34 | 2-(*p*-Chlorophenyl)-5-(phenylthio)pyrrole-3-carbonitrile |
| 35 | 2-(*p*-Chlorophenyl)-1-(ethoxymethyl)-5-(phenylthio)pyrrole-3-carbonitrile |
| 36 | 4-Bromo-2-(*p*-chlorophenyl)-1-(ethoxymethyl)-5-[(*p*-nitrophenyl)thio]pyrrole-3-carbonitrile |
| 37 | 2-(*p*-Chlorophenyl)-5-[(3,4-dichlorophenyl)thio]pyrrole-3-carbonitrile |
| 38 | 2-(*p*-Chlorophenyl)-5-(phenylsulfinyl)pyrrole-3-carbonitrile |
| 39 | 4-Bromo-2-(*p*-chlorophenyl)-5-[(3,4-dichlorophenyl)thio]-1-(ethoxymethyl)pyrrole-3-carbonitrile |
| 40 | 4-Bromo-2-(*p*-chlorophenyl)-1-(ethoxymethyl)-5-(phenylsulfonyl)pyrrole-3-carbonitrile |
| 41 | 4-Bromo-2-(*p*-chlorophenyl)-5-[(3,4-dichlorophenyl)thio]pyrrole-3-carbonitrile |
| 42 | 2-(*p*-Chlorophenyl)-5-[(3,4-dichlorophenyl)thio]-1-(ethoxymethyl)pyrrole-3-carbonitrile |
| 43 | 2-(*p*-Chlorophenyl)-5-[(3,4-dichlorophenyl)sulfinyl]-1-(ethoxymethyl)pyrrole-3-carbonitrile |
| 44 | 5-[(3-Chloro-4-fluorophenyl)thio)-2-(*p*-chlorophenyl)pyrrole-3-carbonitrile |
| 45 | 5-[(3-Chloro-4-fluorophenyl)thio]-2-(*p*-chlorophenyl)-1-(ethoxymethyl)pyrrole-3-carbonitrile |
| 46 | 5-[(3-Chloro-4-fluorophenyl)sulfonyl]-2-(*p*-chlorophenyl)pyrrole-3-carbonitrile |
| 47 | 4-Bromo-5-[(3-chloro-4-fluorophenyl)sulfonyl]-2-(*p*-chlorophenyl)pyrrole-3-carbonitrile |
| 47 | 4-Bromo-5-[(3-chloro-4-fluorophenyl)sulfonyl]-2-(*p*-chlorophenyl)pyrrole-3-carbonitrile |
| 48 | 4-Bromo-5-[(3-chloro-4-fluorophenyl)sulfonyl]-2-(*p*-chlorophenyl)-1-(ethoxymethyl)pyrrole-3-carbonitrile |
| 49 | 5-[(3-Chloro-4-fluorophenyl)sulfinyl]-2-(*p*-chlorophenyl)-1-(ethoxymethyl)pyrrole-3-carbonitrile |
| 50 | 5-[(3-Chloro-4-fluorophenyl)sulfonyl]-2-(*p*-chlorophenyl)-1-(ethoxymethyl)pyrrole-3-carbonitrile |
| 51 | 2-(*p*-Chlorophenyl)-3-(phenylsulfinyl)-5-(trifluoromethyl)pyrrole |
| 52 | 2-(*p*-Chlorophenyl)-3-(phenylthio)-5-(trifluoromethyl)pyrrole |
| 53 | 3-Bromo-5-(*p*-chlorophenyl)-4-(phenylthio)-2-(trifluoromethyl)pyrrole |
| 54 | 3-Chloro-5-(*p*-chlorophenyl)-4-(phenylthio)-2-(trifluoromethyl)pyrrole |
| 55 | 2-(*p*-Chlorophenyl)-3-(phenylsulfonyl)-5-(trifluoromethyl)pyrrole |
| 56 | 3-Bromo-5-(*p*-chlorophenyl)-1-(ethoxymethyl)-4-(phenylthio)-2-(trifluoromethyl)pyrrole |
| 57 | 2-(*p*-Chlorophenyl)-1-(ethoxymethyl)-3-(phenylthio)-5-(trifluoromethyl)pyrrole |
| 58 | 2-(*p*-Chlorophenyl)-1-(ethoxymethyl)-3-(phenylsulfinyl)-5-(trifluoromethyl)pyrrole |
| 59 | 3-[(3-Chloro-4-fluorophenyl)thio]-2-(*p*-chlorophenyl)-5-(trifluoromethyl)pyrrole |
| 60 | 3-Bromo-5-(*p*-chlorophenyl)-4-(phenylsulfonyl)-2-(trifluoromethyl)pyrrole |
| 61 | 3-Bromo-5-(*p*-chlorophenyl)-4-(phenylsulfinyl)-2-(trifluoromethyl)pyrrole |
| 62 | 2-(*p*-Chlorophenyl)-1-(ethoxymethyl)-3-(phenylsulfonyl)-5-(trifluoromethyl)pyrrole |
| 63 | 3-Chloro-5-(p-chlorophenyl)-4-(phenylsulfinyl)-2-(trifluoromethyl)pyrrole |
| 64 | 3-Bromo-4-[(3-chloro-4-fluorophenyl)thio)-5-(*p*-chlorophenyl)-2-(trifluoromethyl)pyrrole |
| 65 | 3-[(3-Chloro-4-fluorophenyl)sulfinyl]-2-(*p*-chlorophenyl)-5-(trifluoromethyl)pyrrole |
| 66 | 4-[(3-Chloro-4-fluorophenyl)sulfinyl]-5-(*p*-chlorophenyl)-2-(trifluoromethyl)pyrrole |
| 67 | 3-Bromo-4-[(3-chloro-4-fluorophenyl)thio]-5-(*p*-chlorophenyl)-1-(ethoxymethyl)-2-(trifluoromethyl)pyrrole |
| 68 | 3-[(3-Chloro-4-fluorophenyl)sulfinyl]-2-(*p*-chlorophenyl)-1-(ethoxymethyl)-5-(trifluoromethyl)pyrrole |
| 69 | 3-[(3-Chloro-4-fluorophenyl)thio]-2-(*p*-chlorophenyl)-1-(ethoxymethyl)-5-(trifluoromethyl)pyrrole |
| 70 | 3-Chloro-5-(*p*-chlorophenyl)-4-(phenylsulfonyl)-2-(trifluoromethyl)pyrrole |
| 71 | 3-Chloro-5-(*p*-chlorophenyl)-1-(ethoxymethyl)-4-(phenylthio)-2-(trifluoromethyl)pyrrole |
| 72 | 3-Bromo-5-(*p*-chlorophenyl)-4-[(*p*-chlorophenyl)sulfinyl]-2-(trifluoromethyl)pyrrole |
| 73 | 2-(*p*-Chlorophenyl)-3-[(*p*-chlorophenyl)sulfonyl]-1-(ethoxymethyl)-5-(trifluoromethyl)pyrrole |
| 74 | 3-Bromo-5-(*p*-chlorophenyl)-4-[(*p*-chlorophenyl)sulfinyl]-1-(ethoxymethyl)-2-(trifluoromethyl)pyrrole |
| 75 | 3-Bromo-4-[(3-chloro-4-fluorophenyl)sulfinyl]-5-(*p*-chlorophenyl)-1-(ethoxymethyl)-2-(trifluoromethyl)pyrrole |
| 76 | 2-(*p*-Chlorophenyl)-3-[(*p*-nitrophenyl)thio]-5-(trifluoromethyl)pyrrole |
| 77 | 2-(*p*-Chlorophenyl)-1-(ethoxymethyl)-3-[(*p*-nitrophenyl)thio]-5-(trifluoromethyl)pyrrole |

**TABLE I**

| **Insecticidal Evaluations** | | | | | |
|---|---|---|---|---|---|
| | **Southern Armyworm Tobacco Budworm** | | | | |
| **Compound Number** | **(ppm) 1000** | **(ppm) 300** | **(ppm) 100** | **(ppm) 300** | **(ppm) 100** |
| 1 | 9 | 9 | 9 | - | 9 |
| 2 | 9 | 9 | 9 | - | 9 |
| 3 | 9 | 9 | 9 | - | 9 |
| 4 | 9 | 9 | 9 | 9 | 9 |
| 5 | 9 | 9 | 9 | 9 | 9 |
| 6 | 9 | 9 | 9 | 9 | 9 |
| 7 | 9 | 9 | 9 | 9 | 9 |
| 8 | 9 | - | 0 | - | 0 |
| 9 | 9 | - | 9 | - | 8 |
| 10 | 9 | - | 9 | - | 9 |
| 11 | 9 | - | 9 | - | 7 |
| 12 | 9 | - | 5 | - | 0 |
| 13 | 9 | 9 | 3 | - | - |
| 14 | 9 | 9 | 9 | 9 | 9 |
| 15 | 9 | 9 | 9 | - | - |
| 16 | 9 | 0 | 0 | - | - |
| 17 | 9 | 9 | 9 | 8 | 8 |
| 18 | 9 | 9 | 9 | - | - |
| 19 | 9 | 9 | 9 | 9 | 9 |
| 20 | 9 | 9 | 9 | 9 | 9 |
| 21 | 9 | 9 | 9 | 9 | 9 |
| 22 | 9 | 9 | 9 | - | - |
| 23 | 9 | 9 | 9 | - | - |
| 24 | 9 | 9 | 9 | - | - |
| 25 | 9 | 9 | 9 | 8 | 8 |
| 26 6 | 9 | 9 | 9 | 9 | 9 |
| 27 | 9 | 9 | 9 | 9 | 9 |
| 28 | 9 | 9 | 9 | 9 | 9 |
| 29 | 2 | - | - | - | - |
| 30 | 0 | - | - | - | - |
| 31 | 5 | - | - | - | - |
| 32 | 3 | - | - | - | - |
| 33 | 8 | 6 | 5 | - | - |
| 34 | 9 | 9 | 9 | 0 | 0 |
| 35 | 9 | 9 | 9 | - | - |
| 36 | 9 | 0 | 0 | - | - |
| 37 | 9 | 9 | 9 | 9 | 9 |
| 38 | 9 | 9 | 9 | - | - |
| 39 | 9 | 9 | 3 | - | - |
| 40 | 9 | 9 | 9 | - | - |
| 41 | 9 | 9 | 9 | - | - |
| 42 | 9 | 9 | 9 | - | - |
| 43 | 9 | 9 | 9 | 9 | 9 |
| 44 | 9 | 9 | 9 | - | - |
| 45 | 9 | 9 | 9 | - | - |
| 46 | 9 | 9 | 9 | - | - |
| 47 | 9 | 9 | 9 | 9 | 9 |
| 48 | 9 | 5 | 5 | - | - |
| 49 | 9 | 9 | 9 | 9 | 9 |
| 50 | 9 | 9 | 9 | 9 | 9 |
| 51 | 7 | 0 | 0 | - | - |
| 52 | 8 | 0 | 0 | 0 | 0 |
| 53 | 9 | 8 | 0 | - | - |
| 54 | 9 | 6 | 4 | 1 | 0 |
| 55 | 9 | 8 | 8 | - | - |
| 56 | 9 | 9 | 6 | 0 | 0 |
| 57 | 3 | - | - | - | - |
| 58 | 6 | - | - | - | - |
| 59 | 4 | - | - | - | - |
| 60 | 9 | 9 | 9 | - | - |
| 61 | 9 | 9 | 9 | 9 | 3 |
| 62 | 9 | 0 | 0 | - | - |
| 63 | 9 | 9 | 9 | 8 | 4 |
| 64 | 9 | 9 | 9 | - | - |
| 65 | 8 | 2 | 2 | 3 | 0 |
| 66 | 9 | 9 | 9 | 9 | 9 |
| 67 | 9 | 9 | 9 | 9 | 9 |
| 68 | 9 | 7 | 7 | 2 | 0 |
| 69 | 9 | 7 | 5 | - | - |
| 70 | 9 | 9 | 9 | 8 | 3 |
| 71 | 9 | 6 | 4 | - | - |
| 72 | 9 | 9 | 9 | - | - |
| 73 | 9 | 9 | 9 | - | - |
| 74 | 9 | 9 | 9 | - | - |
| 75 | 9 | 9 | 9 | - | - |
| 76 | 9 | 8 | 4 | - | - |
| 77 | 6 | - | - | - | - |

## Claims

1. A compound having the structural formula 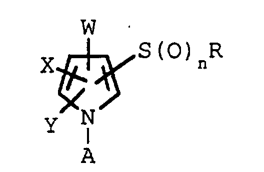 wherein
R and X are each independently phenyl optionally substituted with any combination of from one to five halogen, NO₂, CN, C₁-C₄alkyl, C₁-C₄haloalkyl, C₁-C₄alkoxy or C₁-C₄haloalkoxy groups;
n is an integer of 0, 1 or 2;
W is halogen, CN, NO₂ or C₁-C₄haloalkyl;
Y is hydrogen, halogen or C₁-C₄ haloalkyl;
A is hydrogen, CN, C(O)R₁, CHR₂NHC(O)R₃, CH₂SQ, CHR₄OC(O)(CR₅R₆)mQ₁, C₁-C₆alkyl optionally substituted with
one to three halogen atoms,
one tri(C₁-C₄alkyl)silyl,
one hydroxy,
one cyano,
one or two C₁-C₄alkoxy groups optionally substituted with one to three halogen atoms,
one C₁-C₄alkylthio,
one phenyl optionally substituted with one to three substituents the same or different selected from halogen, C₁-C₄alkyl groups and C₁-C₄alkoxy,
one phenoxy group optionally substituted with one to three substituents the same or different selected from halogen, C₁-C₄alkyl and C₁-C₄alkoxy,
one benzyloxy group optionally substituted on the phenyl ring with one to three substituents the same or different selected from halogen, C₁-C₄alkyl and C₁-C₄alkoxy,
one C₁-C₆alkylcarbonyloxy group optionally substituted with one to three halogen atoms,
one C₂-C₆alkenylcarbonyloxy group optionally substituted with one to three halogen atoms,
one phenylcarbonyloxy group optionally substituted with one to three substituents the same or different selected from halogen, C₁-C₄alkyl and C₁-C₄alkoxy,
one C₁-C₆alkoxycarbonyl group optionally substituted with one to three halogen atoms or one to three C₁-C₄alkoxy groups, or
one benzylcarbonyloxy group optionally substituted on the phenyl ring with one to three substituents the same or different selected from halogen, C₁-C₄alkyl and C₁-C₄alkoxy,
C₃-C₆alkenyl optionally substituted with one to three halogen atoms or one phenyl group, or
C₃-C₆alkynyl optionally substituted with one to three halogen atoms or one phenyl group;
R₁ is C₁-C₆alkyl or C₃-C₆cycloalkyl each optionally substituted with
one to three halogen atoms,
one hydroxy,
one cyano,
one or two C₁-C₄alkoxy groups optionally substituted with one to three halogen atoms,
one C₁-C₄alkylthio,
one phenyl group optionally substituted with one to three substituents the same or different selected from halogen, C₁-C₄alkyl and C₁-C₄alkoxy,
one phenoxy group optionally substituted with one to three substituents the same or different selected from halogen, C₁-C₄alkyl and C₁-C₄alkoxy,
one benzyloxy group optionally substituted on the phenyl ring with one to three substituents the same or different selected from halogen, C₁-C₄alkyl and C₁-C₄alkoxy,
one C₁-C₆alkylcarbonyloxy group optionally substituted with one to three halogen atoms,
one C₂-C₆alkenylcarbonyloxy group optionally substituted with one to three halogen atoms,
one phenylcarbonyloxy group optionally substituted with one to three substituents the same or different selected from halogen, C₁-C₄alkyl and C₁-C₄alkoxy,
one C₁-C₆alkoxycarbonyl group optionally substituted with one to three halogen atoms or one to three C₁-C₄alkoxy groups, or
one benzyloxycarbonyl group optionally substituted on the phenyl ring with one to three substituents the same or different selected from halogen, C₁-C₄alkyl and C₁-C₄alkoxy,
C₂-C₆alkenyl optionally substituted with one to three halogen atoms or one phenyl group,
C₃-C₆alkynyl optionally substituted with one to three halogen atoms or one phenyl group,
phenyl optionally substituted with one or more substituents the same or different selected from halogen, C₁-C₄ alkyl, C₁-C₄alkoxy, phenoxy, C₁-C₄ alkylthio, tri(C₁-C₄ alkyl)silyl, C₁-C₄alkylsulfinyl, C₁-C₄alkylsulfonyl, CN, NO₂ and CF₃ group,
phenoxy optionally substituted with one or more substituents the same or different selected from halogen, C₁-C₄alkyl, C₁-C₄alkoxy, C₁-C₄alkylthio, tri(C₁-C₄alkyl)silyl, C₁-C₄alkylsulfinyl, C₁-C₄alkylsulfonyl, CN groups, NO₂ and CF₃,
1- or 2-naphthyl,
2-, 3- or 4-pyridyl optionally substituted with one to three halogen atoms,
C₁-C₆alkoxy optionally substituted with one to three halogen atoms, or
C₂-C₆alkenyloxy optionally substituted with one to three halogen atoms;
R₂ is hydrogen or C₁-C₄alkyl;
R₃ is C₁-C₆alkyl optionally substituted with one to three halogen atoms,
phenyl optionally substituted with one to three substituents the same or different selected from halogen, CN, NO₂, C₁-C₄alkyl, C₁-C₄alkoxy and CF₃,
2- or 3-thienyl, or
2- or 3-furyl;
Q is 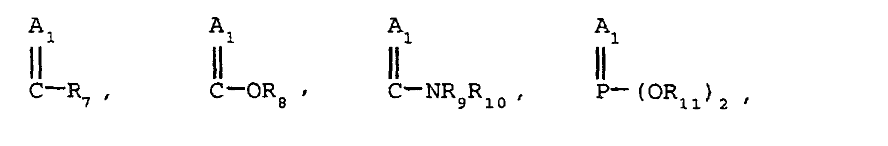 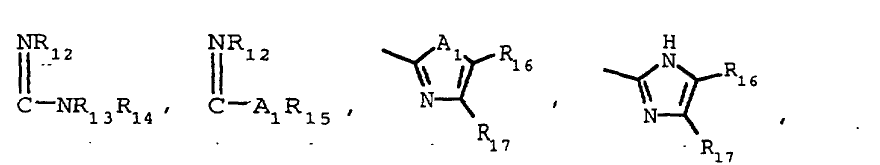 CN,
C₁-C₆alkyl optionally substituted with one or more halogen atoms, CN groups or phenyl groups, or
phenyl optionally substituted with one or more substituents the same or different selected from halogen, C₁-C₄alkyl, C₁-C₄alkoxy, CN, NO₂ groups, CF₃ and NR₁₈R₁₉;
A₁ is O or S;
R₇ is C₁-C₆alkyl or phenyl;
R₈ is C₁-C₆alkyl;
R₉ and R₁₀ are each independently hydrogen, C₁-C₆alkyl or may be taken together with the atom to which they are attached to form a 5- to 7-membered heterocyclic ring;
R₁₁ is C₁-C₄alkyl;
R₁₂ is hydrogen, C₁-C₄alkyl or may be taken together with either R₁₃ or R₁₅ and the atoms to which they are attached to form a 5- to 7-membered heterocyclic ring optionally substituted with one or two C₁-C₄alkyl groups;
R₁₃ and R₁₄ are each independently hydrogen or C₁-C₄alkyl;
R₁₅ is C₁-C₄alkyl or when taken together with R₁₂ and the atoms to which they are attached may form a 5- to 7-membered heterocyclic ring optionally substituted with one or two C₁-C₄alkyl groups;
R₁₆ and R₁₇ are each independently hydrogen or C₁-C₄alkyl or when taken together may form a ring wherein R₁₆R₁₇ is represented by -CH=CH-CH=CH-;
R₁₈ and R₁₉ are each independently hydrogen or C₁-C₄alkyl;
R₄ is hydrogen or C₁-C₄alkyl;
R₅ and R₆ are each independently hydrogen,
C₁-C₆alkyl optionally substituted with one or more halogen atoms,
C₁-C₆alkoxy optionally substituted with one or more halogen atoms,
C₁-C₆alkylthio optionally substituted with one or more halogen atoms, or
phenyl optionally substituted with one or more halogen atoms,
NO₂ groups;
CN groups,
C₁-C₄alkyl groups optionally substituted with one or more halogen atoms, or
C₁-C₄alkoxy groups optionally substituted with one or more halogen atoms, or
when R₅ and R₆ are taken together with the atom to which they are attached may form a C₃-C₆cycloalkyl group optionally substituted with one to three C₁-C₄alkyl groups, C₂-C₆alkenyl groups or phenyl groups, or R₅ or R₆ may be taken together with R₂₀ and the atoms to which they are attached to form a 4- to 7-membered heterocyclic ring;
m is an integer of 0, 1, 2, 3 or 4;
Q₁ is A₂R₂₀, 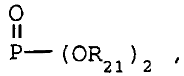 NR₂₂R₂₃, CR₂₄R₂₅C(O)R₂₆, or
C₃-C₆cycloalkyl optionally substituted with one or more C₁-C₆alkyl groups, C₂-C₆alkenyl groups, or
phenyl groups optionally substituted with one or more substituents the same or different selected from halogen atoms,
NO₂ groups,
CN groups,
C₁-C₄alkyl groups optionally substituted with one or more halogen atoms, or
C₁-C₄alkoxy groups optionally substituted with one or more halogen atoms;
A₂ is O or S(O)ₚ;
p is an integer of 0, 1 or 2;
R₂₀ is hydrogen,
C₁-C₆alkyl,
C₂-C₆alkenyl,
C₂-C₆alkynyl,
phenyl optionally substituted with one or more substituents the same or different selected from
halogen atoms,
NO₂ groups,
CN groups,
C₁-C₄alkyl groups optionally substituted with one or more halogen atoms,
C₁-C₄alkoxy groups optionally substituted with one or more halogen atoms,
C(O)R₂₇ provided p is 0,
C(O)R₂₈ provided p is 0,
(CH₂CH₂O)_{q}R₂₇, or 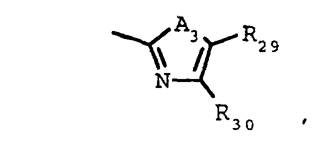 or
R₂₀ may be taken together with either R₅ or R₆ and the atoms to which they are attached to form a 4- to 7-membered heterocyclic ring;
A₃ is O or S;
R₂₇ is C₁-C₆alkyl,
C₂-C₆alkenyl,
C₂-C₆alkynyl, or
phenyl optionally substituted with one or more substituents the same or different selected from
halogen atoms,
NO₂ groups,
CN groups,
C₁-C₄alkyl groups optionally substituted with one or more halogen atoms, or
C₁-C₄alkoxy groups optionally substituted with one or more halogen atoms;
q is an integer of 1, 2 or 3;
R₂₈ is OR₃₁ or NR₃₂R₃₃;
R₃₁ is C₁-C₆alkyl or
phenyl optionally substituted with one or more substituents the same or different selected from
halogen atoms,
NO₂ groups,
CN groups,
C₁-C₄alkyl groups optionally substituted with one or more halogen atoms, or
C₁-C₄alkoxy groups optionally substituted with one or more halogen atoms;
R₃₂ and R₃₃ are each independently hydrogen or C₁-C₄alkyl;
R₂₉ and R₃₀ are each independently hydrogen or C₁-C₄alkyl, or when taken together may form a ring wherein R₂₉R₃₀ is represented by -CH=CH-CH=CH-;
R₂₁ is C₁-C₄alkyl;
R₂₂ is hydrogen,
C₁-C₆alkyl,
C₂-C₆alkenyl,
C₂-C₆alkynyl, or
phenyl optionally substituted with one or more substituents the same or different selected from
halogen atoms,
NO₂ groups,
CN groups,
C₁-C₄alkyl groups optionally substituted with one or more halogen atoms, or
C₁-C₄alkoxy groups optionally substituted with one or more halogen atoms, or
R₂₂ may be taken together with either R₅ or R₆ and the atoms to which they are attached to form a 4- to 7-membered heterocyclic ring;
R₂₃ is hydrogen,
C₁-C₆alkyl,
C₂-C₆alkenyl,
C₂-C₆alkynyl,
phenyl optionally substituted with one or more substituents the same or different selected from
halogen atoms,
NO₂ groups,
CN groups,
C₁-C₄alkyl groups optionally substituted with one or more halogen atoms, or
C₁-C₄alkoxy groups optionally substituted with one or more halogen atoms,
C(A₄)R₃₄,
CN,
SO₂R₃₅, or
C(O)CHR₃₆NHR₃₇;
A₄ is O or S;
R₃₄ is OR₃₈, CO₂R₃₈, NR₃₉R₄₀,
C₁-C₆alkyl optionally substituted with one to three halogen atoms,
C₂-C₆alkenyl,
C₂-C₆alkynyl, or
phenyl optionally substituted with one or more substituents the same or different selected from
halogen atoms,
NO₂ groups,
CN groups,
C₁-C₄alkyl groups optionally substituted with one or more halogen atoms, or
C₁-C₄alkoxy groups optionally substituted with one or more halogen atoms;
R₃₈ is C₁-C₆alkyl optionally substituted with one phenyl group, or phenyl optionally substituted with one or more substituents the same or different selected from
halogen atoms,
NO₂ groups,
CN groups,
C₁-C₄alkyl groups optionally substituted with one or more halogen atoms, or
C₁-C₄alkoxy groups optionally substituted with one or more halogen atoms;
R₃₉ and R₄₀ are each independently hydrogen or C₁-C₄alkyl;
R₃₅ is NR₄₁R₄₂,
C₁-C₆alkyl,
C₂-C₆alkenyl,
C₂-C₆alkynyl, or
phenyl optionally substituted with one or more substituents the same or different selected from
halogen atoms,
NO₂ groups,
CN groups,
C₁-C₄alkyl groups optionally substituted with one or more halogen atoms, or
C₁-C₄alkoxy groups optionally substituted with one or more halogen atoms;
R₄₁ and R₄₂ are each independently hydrogen or C₁-C₄alkyl;
R₃₆ is hydrogen,
C₁-C₄alkyl optionally substituted with
one hydroxy group,
one SR₄₃ group,
one C(O)NH₂ group,
one NH₂ group,
one NHC(=NH)NH₂ group,
one CO₂H group,
one phenyl group optionally substituted with one hydroxy group,
one 3-indolyl group or
one 4-imidazolyl group;
R₄₃ is hydrogen or C₁-C₄alkyl;
R₃₇ is C(A₄)R₄₄;
R₄₄ is C₁-C₆alkyl optionally substituted with one or more halogen atoms,
C₂-C₆alkoxyalkyl,
C₁-C₆alkylthio,
phenyl optionally substituted with one or more substituents the same or different selected from
halogen atoms,
NO₂ groups,
CN groups,
C₁-C₄alkyl groups optionally substituted with one or more halogen atoms, or
C₁-C₄alkoxy groups optionally substituted with one or more halogen atoms,
OR₃₈,
CO₂R₃₈ or
NR₃₉R₄₀;
R₂₄ and R₂₅ are each independently hydrogen,
C₁-C₆alkyl optionally substituted with one or more halogen atoms,
C₁-C₆alkoxy optionally substituted with one or more halogen atoms,
C₁-C₆alkylthio optionally substituted with one or more halogen atoms,
phenyl optionally substituted with one or more substituents the same or different selected from
halogen atoms,
CN groups,
NO₂ groups,
C₁-C₄alkyl groups optionally substituted with one or more halogen atoms, or
C₁-C₄alkoxy groups optionally substituted with one or more halogen atoms, or
when R₂₄ and R₂₅ are taken together with the atom to which they are attached may form a C₃-C₆cycloalkyl group optionally substituted with one to three C₁-C₄alkyl groups, C₂-C₆alkenyl groups or phenyl groups;
R₂₆ is OR₄₅, NR₄₁R₄₂, C₁-C₄alkyl or
phenyl optionally substituted with one or more substituents the same or different selected from
halogen atoms,
CN groups,
NO₂ groups,
C₁-C₄alkyl groups optionally substituted with one or more halogen atoms, or
C₁-C₄alkoxy groups optionally substituted with one or more halogen atoms; and
R₄₅ is C₁-C₄alkyl or
phenyl optionally substituted with one or more substituents the same or different selected from
halogen atoms,
CN groups,
NO₂ groups,
C₁-C₄alkyl groups optionally substituted with one or more halogen atoms, or
C₁-C₄alkoxy groups optionally substituted with one or more halogen atoms.

2. The compound according to claim 1 wherein
R and X are each independently phenyl optionally substituted with any combination of from one to three halogen, NO₂, CN, C₁-C₄alkyl, C₁-C₄haloalkyl, C₁-C₄alkoxy or C₁-C₄haloalkoxy groups;
n is an integer of 0, 1 or 2;
W is Cl, Br, CN, NO₂ or C₁-C₄haloalkyl;
Y is hydrogen, Cl, Br or C₁-C₄haloalkyl;
A is hydrogen, CN, C(O)R₁ or
C₁-C₄alkyl optionally substituted with
one to three halogen atoms,
one cyano,
one C₁-C₄alkoxy group,
one C₁-C₆alkylcarbonyloxy group,
one phenylcarbonyloxy group optionally substituted with one to three halogen atoms or one C₁-C₄alkyl group, or
one benzylcarbonyloxy group; and
R₁ is phenyl optionally substituted with one to three halogen atoms, one or two C₁-C₄alkyl groups, one or two C₁-C₄alkoxy groups, one CN group, one NO₂ group or one CF₃ group.

3. The compound according to claim 2 selected from the group consisting of
2-(*p*-chlorophenyl)-4-[(3,4-dichlorophenyl)thio]-5-(trifluoromethyl)pyrrole-3-carbonitrile;
2-(*p*-chlorophenyl)-4-[(*p*-chlorophenyl)thio]-5-(trifluoromethyl)pyrrole-3-carbonitrile;
3-bromo-4-[(3-chloro-4-fluorophenyl)sulfonyl]-5-(*p*-chlorophenyl)-2-(trifluoromethyl)pyrrole;
3-bromo-5-(*p*-chlorophenyl)-4-[(*p*-chlorophenyl)sulfonyl]-2-(trifluoromethyl)pyrrole;
4-bromo-2-(*p*-chlorophenyl)-5-[(*p*-chlorophenyl)sulfonyl]pyrrole-3-carbonitrile;
2-(*p*-chlorophenyl)-5-[(3,4-dichlorophenyl)sulfonyl]pyrrole-3-carbonitrile; and
2-(*p*-chlorophenyl)-5-[(3,4-dichlorophenyl)sulfonyl]-1-(ethoxymethyl)pyrrole-3-carbonitrile.

4. A method for controlling insects which comprises contacting said insects, their breeding grounds, food supply or habitat with an insecticidally effective amount of a compound having the structural formula 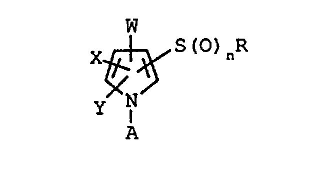 wherein A, R, W, X, Y and n are as described in claim 1.

5. The method according to claim 4 wherein the compound is selected from the group consisting of 2-(*p*-chlorophenyl)-4-[(3,4-dichlorophenyl)thio]-5-(trifluoromethyl)pyrrole-3-carbonitrile;
2-(*p*-chlorophenyl)-4-[(*p*-chlorophenyl)thio]-5-(trifluoromethyl)pyrrole-3-carbonitrile;
3-bromo-4-[(3-chloro-4-fluorophenyl)sulfonyl]-5-(*p*-chlorophenyl)-2-(trifluoromethyl)pyrrole;
3-bromo-5-(*p*-chlorophenyl)-4-[(*p*-chlorophenyl)sulfonyl]-2-(trifluoromethyl)pyrrole;
4-bromo-2-(*p*-chlorophenyl)-5-[(*p*-chlorophenyl)sulfonyl]pyrrole-3-carbonitrile;
2-(*p*-chlorophenyl)-5-[(3,4-dichlorophenyl)sulfonyl]pyrrole-3-carbonitrile; and
2-(*p*-chlorophenyl)-5-[(3,4-dichlorophenyl)sulfonyl]-1-(ethoxymethyl)pyrrole-3-carbonitrile.

6. A method for protecting growing plants from attack by insects which comprises applying to the foliage of said plants or to the soil or water in which they are growing an insecticidally effective amount of a compound having the structural formula 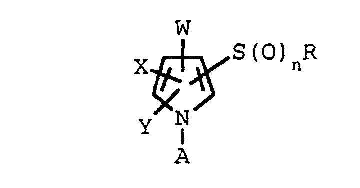 wherein A, R, W, X, Y and n are as described in claim 1.

7. The method according to claim 6 wherein the compound is selected from the group consisting of
2-(*p*-chlorophenyl)-4-[(3,4-dichlorophenyl)thio]-5-(trifluoromethyl)pyrrole-3-carbonitrile;
2-(*p*-chlorophenyl)-4-[(*p*-chlorophenyl)thio]-5-(trifluoromethyl)pyrrole-3-carbonitrile;
3-bromo-4-[(3-chloro-4-fluorophenyl)sulfonyl]-5-(*p*-chlorophenyl)-2-(trifluoromethyl)pyrrole;
3-bromo-5-(*p*-chlorophenyl)-4-[(*p*-chlorophenyl)sulfonyl]-2-(trifluoromethyl)pyrrole;
4-bromo-2-(*p*-chlorophenyl)-5-[(*p*-chlorophenyl)sulfonyl]pyrrole-3-carbonitrile;
2-(*p*-chlorophenyl)-5-[(3,4-dichlorophenyl)sulfonyl]pyrrole-3-carbonitrile; and
2-(*p*-chlorophenyl)-5-[(3,4-dichlorophenyl)sulfonyl]-1-(ethoxymethyl)pyrrole-3-carbonitrile.

8. The method according to claim 6 wherein the compound is applied to the plants or to the soil or water in which they are growing at a rate of about 0.1 kg/ha to 4.0 kg/ha.

9. A composition for controlling insects which comprises an agronomically acceptable carrier and an insecticidally effective amount of a compound having the structural formula 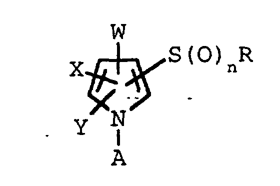 wherein A, R, W, X, Y and n are as described in claim 1.

10. The composition according to claim 9 wherein A, R, W, X, Y and n are as described in claim 2.

11. A process for preparing a compound of formula I as defined in Claim 1 which comprises one of the following:
a) reacting a compound of formula 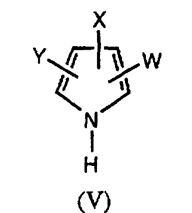 wherein W, X and Y are as defined in Claim 1 with a compound of formula
**RSZ** (V1)
wherein R is as defined in Claim 1 and Z is Cl or Br to give a compound of formula I wherein A is hydrogen and n is 0, if desired oxidising with an oxidising agent to give a corresponding compound of formula I wherein n is 1 or 2;
or
b) reacting a compound of formula I wherein A is hydrogen with an alkylating, alkenylating, alkynylating agent of formula:
**A halide**
wherein A is C₁-C₆ alkyl, C₃-C₆ alkenyl or C₃-C₆ alkynyl each optionally substituted as defined in Claim 1 to give a corresponding compound of formula I;
or
c) acylating a compound of formula I as defined in Claim 1 wherein A is hydrogen with an acyl chloride of formula
**R**_{**1**}**COCl**
wherein R₁ is as defined in Claim 1 to give a corresponding compound of formula I where A is COR₁ ;
or
d) reacting a compound of formula I as defined in Claim 1 wherein A is CH₂Cl with a compound of formula
**(alkali metal) SQ**
wherein Q is as defined in Claim 1 to give a corresponding compound of formula I wherein A is SQ;
or
e) reacting a compound of formula I as defined in Claim 1 wherein A is H with a compound of formula
**R**_{**3**}**C(O)NHCH(R**_{**2**}**)OC(O)CH**_{**3**}
wherein R₂ and R₃ are as defined in Claim 1 to give a corresponding compound of formula I wherein A is CHR₂NHC(O)R₃;
or
f) reacting a compound of formula I as defined in Claim 1 wherein A is CHR₂NHC(O)R₃ with a phosphorus oxyhalide (such as POCl₃) to give a corresponding compound of formula I where A represents -CHR₂ halogen (i.e when R₂ = hydrogen, A = halomethyl and when R₂ = C₁-C₄ alkyl, A represents α- halo(C₂-C₅)alkyl);
or
g) reacting a compound of formula I as defined in Claim 1 wherein A is -CHR₂ halogen with a compound of formula:
**Q**_{**1**}**(CR**_{**5**}**R**_{**6**}**)**_{**m**}**CO**_{**2**}**H**
wherein m, R₅, R₆ and Q are as defined in Claim 1 to give a corresponding compound of formula I wherein A is
**-CHR**_{**4**}**OC(O)(CR**_{**5**}**R**_{**6**}**)**_{**m**}**Q**_{**1**}
as defined in Claim 1.

## Patentansprüche

1. Verbindung der Strukturformel 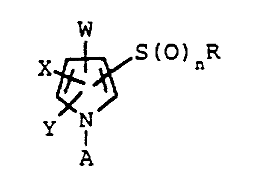 in der
R und X jeweils unabhängig voneinander gegebenenfalls mit einer beliebigen Kombination von einem bis fünf Halogenatomen bzw. einer bis fünf NO₂-, CN-, C₁-C₄Alkyl-, C₁-C₄Halogenalkyl-, C₁-C₄Alkoxy- oder C₁-C₄Halogenalkoxygruppen substituiertes Phenyl bedeuten;
n eine ganze Zahl 0, 1 oder 2 bedeutet;
W Halogen, CN, NO₂ oder C₁-C₄Halogenalkyl bedeutet;
Y Wasserstoff, Halogen oder C₁-C₄Halogenalkyl bedeutet;
A Wasserstoff, CN, C(O)R₁, CHR₂NHC(O)R₃, CH₂SQ, CHR₄OC(O) (CR₅R₆)ₘQ₁, C₁-C₆Alkyl, das gegebenenfalls substituiert ist durch:
ein bis drei Halogenatome,
eine Tri(C₁-C₄alkyl)silylgruppe,
eine Hydroxygruppe,
eine Cyangruppe,
eine oder zwei C₁-C₄Alkoxygruppen, die gegebenenfalls durch ein bis drei Halogenatome substituiert sind,
eine C₁-C₄Alkylthiogruppe,
eine Phenylgruppe, die gegebenenfalls durch einen bis drei gleiche oder verschiedene Substituenten aus der Gruppe Halogen, C₁-C₄Alkyl und C₁-C₄Alkoxy substituiert ist,
eine Phenoxygruppe, die gegebenenfalls durch einen bis drei gleiche oder verschiedene Substituenten aus der Gruppe Halogen, C₁-C₄Alkyl und C₁-C₄Alkoxy substituiert ist,
eine Benzyloxygruppe, die gegebenenfalls am Phenylring durch einen bis drei gleiche oder verschiedene Substituenten aus der Gruppe Halogen, C₁-C₄Alkyl und C₁-C₄Alkoxy substituiert ist,
eine C₁-C₆Alkylcarbonyloxygruppe, die gegebenenfalls durch ein bis drei Halogenatome substituiert ist,
eine C₂-C₆Alkenylcarbonyloxygruppe, die gegebenenfalls durch ein bis drei Halogenatome substituiert ist,
eine Phenylcarbonyloxygruppe, die gegebenenfalls durch einen bis drei gleiche oder verschiedene Substituenten aus der Gruppe Halogen, C₁-C₄Alkyl und C₁-C₄Alkoxy substituiert ist,
eine C₁-C₆Alkoxycarbonylgruppe, die gegebenenfalls durch ein bis drei Halogenatome oder eine bis drei C₁-C₄Alkoxygruppen substituiert ist, oder
eine Benzylcarbonyloxygruppe, die gegebenenfalls am Phenylring durch einen bis drei gleiche oder verschiedene Substituenten aus der Gruppe Halogen, C₁-C₄Alkyl und C₁-C₄Alkoxy substituiert ist,
C₃-C₆Alkenyl, das gegebenenfalls durch ein bis drei Halogenatome oder eine Phenylgruppe substituiert ist, oder
C₃-C₆Alkinyl, das gegebenenfalls durch ein bis drei Halogenatome oder eine Phenylgruppe substituiert ist, bedeutet; dabei haben die Symbole die folgende Bedeutung:
R₁ bedeutet C₁-C₆Alkyl oder C₃-C₆Cycloalkyl, die jeweils gegebenenfalls substituiert sind durch:
ein bis drei Halogenatome,
eine Hydroxygruppe,
eine Cyangruppe,
eine oder zwei C₁-C₄Alkoxygruppen, die gegebenenfalls durch ein bis drei Halogenatome substituiert sind,
eine C₁-C₄Alkylthiogruppe,
eine Phenylgruppe, die gegebenenfalls durch einen bis drei gleiche oder verschiedene Substituenten aus der Gruppe Halogen, C₁-C₄Alkyl und C₁-C₄Alkoxy substituiert ist,
eine Phenoxygruppe, die gegebenenfalls durch einen bis drei gleiche oder verschiedene Substituenten aus der Gruppe Halogen, C₁-C₄Alkyl und C₁-C₄Alkoxy substituiert ist,
eine Benzyloxygruppe, die gegebenenfalls am Phenylring durch einen bis drei gleiche oder verschiedene Substituenten aus der Gruppe Halogen, C₁-C₄Alkyl und C₁-C₄Alkoxy substituiert ist,
eine C₁-C₆Alkylcarbonyloxygruppe, die gegebenenfalls durch ein bis drei Halogenatome substituiert ist,
eine C₂-C₆Alkenylcarbonyloxygruppe, die gegebenenfalls durch ein bis drei Halogenatome substituiert ist,
eine Phenylcarbonyloxygruppe, die gegebenenfalls durch einen bis drei gleiche oder verschiedene Substituenten aus der Gruppe Halogen, C₁-C₄Alkyl und C₁-C₄Alkoxy substituiert ist,
eine C₁-C₆Alkoxycarbonylgruppe, die gegebenenfalls durch ein bis drei Halogenatome oder eine bis drei C₁-C₄Alkoxygruppen substituiert ist, oder
eine Benzyloxycarbonylgruppe, die gegebenenfalls am Phenylring durch einen bis drei gleiche oder verschiedene Substituenten aus der Gruppe Halogen, C₁-C₄Alkyl und C₁-C₄Alkoxy substituiert ist,
C₂-C₆Alkenyl, das gegebenenfalls durch ein bis drei Halogenatome oder eine Phenylgruppe substituiert ist, oder
C₃-C₆Alkinyl, das gegebenenfalls durch ein bis drei Halogenatome oder eine Phenylgruppe substituiert ist,
Phenyl, das gegebenenfalls durch einen oder mehrere gleiche oder verschiedene Substituenten aus der Gruppe Halogen, C₁-C₄Alkyl, C₁-C₄Alkoxy, Phenoxy, C₁₋C₄Alkylthio, Tri (C₁-C₄alkyl)silyl; C₁₋C₄Alkylsulfinyl, C₁-C₄Alkylsulfonyl, CN, NO₂ und CF₃ substituiert ist,
Phenoxy, das gegebenenfalls durch einen oder mehrere gleiche oder verschiedene Substituenten aus der Gruppe Halogen, C₁-C₄Alkyl, C₁-C₄Alkoxy, C₁-C₄Alkylthio, Tri (C₁₋C₄alkyl)silyl, C₁₋C₄Alkylsulfinyl, C₁-C₄Alkylsulfonyl, CN, NO₂ und CF₃ substituiert ist,
1- oder 2-Naphthyl,
2-, 3- oder 4-Pyridyl, das gegebenenfalls durch ein bis drei Halogenatome substituiert ist,
C₁-C₆Alkoxy, das gegebenenfalls durch ein bis drei Halogenatome substituiert ist, oder
C₂-C₆Alkenyloxy, das gegebenenfalls durch ein bis drei Halogenatome substituiert ist;
R₂ bedeutet Wasserstoff oder C₁-C₄Alkyl;
R₃ bedeutet C₁-C₆Alkyl, das gegebenenfalls durch ein bis drei Halogenatome substituiert ist,
Phenyl, das gegebenenfalls durch einen bis drei gleiche oder verschiedene Substituenten aus der Gruppe Halogen, CN, NO₂, C₁-C₄Alkyl, C₁-C₄Alkoxy und CF₃ substituiert ist,
2- oder 3-Thienyl, oder
2- oder 3-Furyl;
Q bedeutet 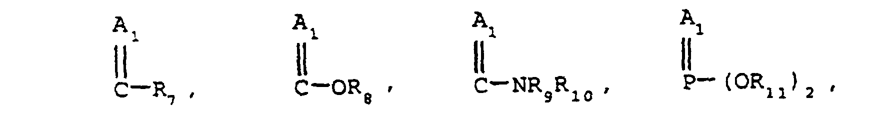 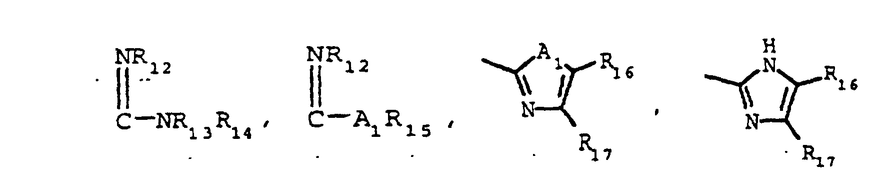 CN,
C₁-C₆Alkyl, das gegebenenfalls durch ein oder mehrere Halogenatome, CN-Gruppen oder Phenylgruppen substituiert ist, oder
Phenyl, das gegebenenfalls durch einen oder mehrere gleiche oder verschiedene Substituenten aus der Gruppe Halogen, C₁-C₄Alkyl, C₁-C₄Alkoxy, CN, NO₂, CF₃ und NR₁₈R₁₉ substituiert ist;
A₁ bedeutet 0 oder S;
R₇ bedeutet C₁-C₆Alkyl oder Phenyl;
R₈ bedeutet C₁-C₆Alkyl;
R₉ und R₁₀ bedeuten jeweils unabhängig voneinander Wasserstoff, C₁-C₆Alkyl oder bilden gemeinsam mit dem Atom, an das sie gebunden sind, einen 5- bis 7-gliedrigen Heterocyclus;
R₁₁ bedeutet C₁-C₄Alkyl;
R₁₂ bedeutet Wasserstoff, C₁-C₄Alkyl oder bildet gemeinsam mit R₁₃ oder R₁₅ und den Atomen, an die sie gebunden sind, einen 5- bis 7-gliedrigen Heterocyclus, der gegebenenfalls durch eine oder zwei C₁-C₄Alkylgruppen substituiert ist;
R₁₃ und R₁₄ bedeuten jeweils unabhängig voneinander Wasserstoff oder C₁-C₄Alkyl;
R₁₅ bedeutet C₁-C₄Alkyl oder kann gemeinsam mit R₁₂ und den Atomen, an die sie gebunden sind, einen 5- bis 7-gliedrigen Heterocyclus bilden, der gegebenenfalls durch eine oder zwei C₁-C₄Alkylgruppen substituiert ist;
R₁₆ und R₁₇ bedeuten jeweils unabhängig voneinander Wasserstoff oder C₁-C₄Alkyl oder können gemeinsam einen Ring bilden, in dem R₁₆R₁₇ -CH=CH-CH=CH- darstellt;
R₁₈ und R₁₉ bedeuten jeweils unabhängig voneinander Wasserstoff oder C₁-C₄Alkyl;
R₄ bedeutet Wasserstoff oder C₁-C₄Alkyl;
R₅ und R₆ bedeuten jeweils unabhängig voneinander Wasserstoff,
C₁-C₆Alkyl, das gegebenenfalls durch ein oder mehrere Halogenatome substituiert ist,
C₁-C₆Alkoxy, das gegebenenfalls durch ein oder mehrere Halogenatome substituiert ist,
C₁-C₆Alkylthio, das gegebenenfalls durch ein oder mehrere Halogenatome substituiert ist, oder
Phenyl, das gegebenenfalls substituiert ist durch ein oder mehrere
Halogenatome;
NO₂-Gruppen;
CN-Gruppen;
C₁-C₄Alkylgruppen, die gegebenenfalls durch ein oder mehrere Halogenatome substituiert sind, oder
C₁-C₄Alkoxygruppen, die gegebenenfalls durch ein oder mehrere Halogenatome substituiert sind, oder
R₅ und R₆ können gemeinsam mit dem Atom, an das sie gebunden sind, eine C₃-C₆Cycloalkylgruppe bilden, die gegebenenfalls durch eine bis drei C₁-C₄Alkylgruppen, C₂-C₆Alkenylgruppen oder Phenylgruppen substituiert ist, oder R₅ und R₆ können gemeinsam mit R₂₀ und den Atomen, an die sie gebunden sind, einen 4- bis 7-gliedrigen Heterocyclus bilden;
m bedeutet eine ganze Zahl 0, 1, 2, 3 oder 4;
Q₁ bedeutet A₂R₂₀, 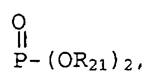 NR₂₂R₂₃, CR₂₄R₂₅C(O)R₂₆, oder C₃-C₆Cycloalkyl, das gegebenenfalls substituiert ist durch: eine oder mehrere C₁-C₆Alkylgruppen, C₂-C₆Alkenylgruppen, oder
Phenylgruppen, die gegebenenfalls substituiert sind durch einen oder mehrere gleiche oder verschiedene Substituenten aus der Gruppe:
Halogenatome,
NO₂-Gruppen,
CN-Gruppen,
C₁-C₄Alkylgruppen, die gegebenenfalls durch ein oder mehrere Halogenatome substituiert sind, oder
C₁-C₄Alkoxygruppen, die gegebenenfalls durch ein oder mehrere Halogenatome substituiert sind;
A₂ bedeutet O oder S(O)ₚ;
p bedeutet eine ganze Zahl 0, 1 oder 2;
R₂₀ bedeutet Wasserstoff,
C₁-C₆Alkyl,
C₂-C₆Alkenyl,
C₂-C₆Alkinyl,
Phenyl,
das gegebenenfalls substituiert ist durch einen oder mehrere gleiche oder verschiedene Substituenten aus der Reihe:
Halogenatome,
NO₂-Gruppen,
CN-Gruppen,
C₁-C₄Alkylgruppen, die gegebenenfalls durch ein oder mehrere Halogenatome substituiert sind,
C₁-C₄Alkoxygruppen, die gegebenenfalls durch ein oder mehrere Halogenatome substituiert sind,
C(O)R₂₇, mit der Maßgabe, daß p 0 bedeutet,
C(O)R₂₈, mit der Maßgabe, daß p 0 bedeutet,
(CH₂CH₂O)_{q}R₂₇, oder 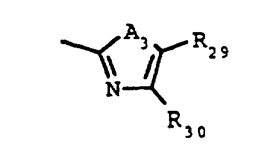 oder
R₂₀ kann gemeinsam mit R₅ oder R₆ und den Atomen, an die sie gebunden sind, einen 4- bis 7-gliedrigen Heterocyclus bilden;
A₃ bedeutet 0 oder S;
R₂₇ bedeutet C₁-C₆Alkyl,
C₂-C₆Alkenyl,
C₂-C₆Alkinyl, oder
Phenyl, das gegebenenfalls substituiert ist durch einen oder mehrere gleiche oder verschiedene Substituenten aus der Reihe:
Halogenatome,
NO₂-Gruppen,
CN-Gruppen,
C₁-C₄Alkylgruppen, die gegebenenfalls durch ein oder mehrere Halogenatome substituiert sind, oder
C₁-C₄Alkoxygruppen, die gegebenenfalls durch ein oder mehrere Halogenatome substituiert sind;
q bedeutet eine ganze Zahl 1, 2 oder 3;
R₂₈ bedeutet OR₃₁ oder NR₃₂R₃₃;
R₃₁ bedeutet C₁-C₆Alkyl oder
Phenyl, das gegebenenfalls substituiert ist durch einen oder mehrere gleiche oder verschiedene Substituenten aus der Reihe:
Halogenatome,
NO₂-Gruppen,
CN-Gruppen,
C₁-C₄Alkylgruppen, die gegebenenfalls durch ein oder mehrere Halogenatome substituiert sind, oder
C₁-C₄Alkoxygruppen, die gegebenenfalls durch ein oder mehrere Halogenatome substituiert sind;
R₃₂ und R₃₃ bedeuten jeweils unabhängig voneinander Wasserstoff oder C₁-C₄Alkyl;
R₂₉ und R₃₀ bedeuten jeweils unabhängig voneinander Wasserstoff oder C₁-C₄Alkyl oder können gemeinsam einen Ring bilden, bei dem R₂₉R₃₀ -CH=CH-CH=CH- darstellt;
R₂₁ bedeutet C₁-C₄Alkyl;
R₂₂ bedeutet Wasserstoff,
C₁-C₆Alkyl,
C₂-C₆Alkenyl,
C₂-C₆Alkinyl, oder
Phenyl, das gegebenenfalls substituiert ist durch einen oder mehrere gleiche oder verschiedene Substituenten aus der Reihe:
Halogenatome,
NO₂-Gruppen,
CN-Gruppen,
C₁-C₄Alkylgruppen, die gegebenenfalls durch ein oder mehrere Halogenatome substituiert sind, oder
C₁-C₄Alkoxygruppen, die gegebenenfalls durch ein oder mehrere Halogenatome substituiert sind, oder
R₂₂ kann gemeinsam mit R₅ oder R₆ und den Atomen, an die sie gebunden sind, einen 4- bis 7-gliedrigen Heterocyclus bilden;
R₂₃ bedeutet Wasserstoff,
C₁-C₆Alkyl,
C₂-C₆Alkenyl,
C₂-C₆Alkinyl,
Phenyl, das gegebenenfalls substituiert ist durch einen oder mehrere gleiche oder verschiedene Substituenten aus der Reihe:
Halogenatome,
NO₂-Gruppen,
CN-Gruppen,
C₁-C₄Alkylgruppen, die gegebenenfalls durch ein oder mehrere Halogenatome substituiert sind, oder
C₁-C₄Alkoxygruppen, die gegebenenfalls durch ein oder mehrere Halogenatome substituiert sind,
C(A₄)R₃₄,
CN,
SO₂R₃₅ oder
C(O)CHR₃₆NHR₃₇;
A₄ bedeutet 0 oder S;
R₃₄ bedeutet OR₃₈, CO₂R₃₈, NR₃₉R₄₀,
C₁-C₆Alkyl, das gegebenenfalls durch ein bis drei Halogenatome substituiert ist,
C₂-C₆Alkenyl,
C₂-C₆Alkinyl, oder
Phenyl, das gegebenenfalls substituiert ist durch einen oder mehrere gleiche oder verschiedene Substituenten aus der Gruppe
Halogenatome,
NO₂-Gruppen,
CN-Gruppen,
C₁-C₄Alkylgruppen, die gegebenenfalls durch ein oder mehrere Halogenatome substituiert sind oder
C₁-C₄Alkoxygruppen, die gegebenenfalls durch ein oder mehrere Halogenatome substituiert sind;
R₃₈ bedeutet C₁-C₆Alkyl, das gegebenenfalls durch
eine Phenylgruppe substituiert ist, oder Phenyl, das gegebenenfalls substituiert ist durch einen oder mehrere gleiche oder verschiedene Substituenten aus der Gruppe
Halogenatome,
NO₂-Gruppen,
CN-Gruppen,
C₁-C₄Alkylgruppen, die gegebenenfalls durch ein oder mehrere Halogenatome substituiert sind oder
C₁-C₄Alkoxygruppen, die gegebenenfalls durch ein oder mehrere Halogenatome substituiert sind;
R₃₉ und R₄₀ bedeuten jeweils unabhängig voneinander Wasserstoff oder C₁-C₄Alkyl;
R₃₅ bedeutet NR₄₁R₄₂,
C₁-C₆Alkyl,
C₂-C₆Alkenyl,
C₂-C₆Alkinyl, oder
Phenyl, das gegebenenfalls substituiert ist durch einen oder mehrere gleiche oder verschiedene Substituenten aus der Reihe:
Halogenatome,
NO₂-Gruppen,
CN-Gruppen,
C₁-C₄Alkylgruppen, die gegebenenfalls durch ein oder mehrere Halogenatome substituiert sind, oder
C₁-C₄Alkoxygruppen, die gegebenenfalls durch ein oder mehrere Halogenatome substituiert sind;
R₄₁ und R₄₂ bedeuten jeweils unabhängig voneinander Wasserstoff oder C₁-C₄Alkyl;
R₃₆ bedeutet Wasserstoff,
C₁-C₄Alkyl, das gegebenenfalls substituiert ist durch
eine Hydroxygruppe,
eine SR₄₃-Gruppe,
eine C(O)NH₂-Gruppe,
eine NH₂-Gruppe,
eine NHC(=NH)NH₂-Gruppe,
eine CO₂H-Gruppe,
eine Phenylgruppe, die gegebenenfalls durch eine Hydroxygruppe substituiert ist,
eine 3-Indolylgruppe oder
eine 4-Imidazolylgruppe;
R₄₃ bedeutet Wasserstoff oder C₁-C₄Alkyl;
R₃₇ bedeutet C(A₄)R₄₄;
R₄₄ bedeutet C₁-C₆Alkyl, das gegebenenfalls durch
ein oder mehrere Halogenatome substituiert ist,
C₂-C₆Alkoxyalkyl,
C₁-C₆Alkylthio,
Phenyl, das gegebenenfalls substituiert ist durch einen oder mehrere gleiche oder verschiedene Substituenten aus der Gruppe
Halogenatome,
NO₂-Gruppen,
CN-Gruppen,
C₁-C₄Alkylgruppen, die gegebenenfalls durch ein oder mehrere Halogenatome substituiert sind oder
C₁-C₄Alkoxygruppen, die gegebenenfalls durch ein oder mehrere Halogenatome substituiert sind
OR₃₈,
CO₂R₃₈ oder
NR₃₉R₄₀;
R₂₄ und R₂₅ bedeuten jeweils unabhängig voneinander Wasserstoff,
C₁-C₆Alkyl, das gegebenenfalls durch ein oder mehrere Halogenatome substituiert ist,
C₁-C₆Alkoxy, das gegebenenfalls durch ein oder mehrere Halogenatome substituiert ist,
C₁-C₆Alkylthio, das gegebenenfalls durch ein oder mehrere Halogenatome substituiert ist,
Phenyl, das gegebenenfalls substituiert ist durch einen oder mehrere gleiche oder verschiedene Substituenten aus der Gruppe
Halogenatome;
CN-Gruppen;
NO₂-Gruppen;
C₁-C₄Alkylgruppen, die gegebenenfalls durch ein oder mehrere Halogenatome substituiert sind, oder
C₁-C₄Alkoxygruppen, die gegebenenfalls durch ein oder mehrere Halogenatome substituiert
sind, oder
R₂₇ und R₂₅ können gemeinsam mit dem Atom, an das sie gebunden sind, eine C₃-C₆Cycloalkylgruppe bilden, die gegebenenfalls durch eine bis drei C₁-C₄Alkylgruppen, C₂-C₆Alkenylgruppen oder Phenylgruppen substituiert ist;
R₂₆ bedeutet OR₄₅, NR₄₁R₄₂, C₁-C₄Alkyl oder
Phenyl, das gegebenenfalls substituiert ist durch einen oder mehrere gleiche oder verschiedene Substituenten aus der
Gruppe
Halogenatome,
CN-Gruppen,
NO₂-Gruppen,
C₁-C₄Alkylgruppen, die gegebenenfalls durch ein oder mehrere Halogenatome substituiert sind oder
C₁-C₄Alkoxygruppen, die gegebenenfalls durch ein oder mehrere Halogenatome substituiert sind; und
R₄₅ bedeutet C₁-C₄Alkyl oder
Phenyl, das gegebenenfalls substituiert ist durch einen oder mehrere gleiche oder verschiedene Substituenten aus der
Gruppe
Halogenatome,
CN-Gruppen,
NO₂-Gruppen,
C₁-C₄Alkylgruppen, die gegebenenfalls durch ein oder mehrere Halogenatome substituiert sind oder
C₁-C₄Alkoxygruppen, die gegebenenfalls durch ein oder mehrere Halogenatome substituiert sind.

2. Verbindung nach Anspruch 1, bei der
R und X jeweils unabhängig voneinander Phenyl bedeuten, das gegebenenfalls durch eine beliebige Kombination von einem bis drei Halogenatomen bzw, einer bis drei NO₂-, CN-, C₁-C₄Alkyl-, C₁-C₄Halogenalkyl-, C₁-C₄Alkoxy- oder C₁-C₄Halogenalkoxygruppen substituiert ist;
n eine ganze Zahl 0, 1 oder 2 bedeutet;
W Cl, Br, CN, NO₂ oder C₁-C₄Halogenalkyl bedeutet;
Y Wasserstoff, Cl, Br oder C₁-C₄Halogenalkyl bedeutet;
A Wasserstoff, CN, C(O)R₁ oder
C₁-C₄Alkyl bedeutet, das gegebenenfalls substituiert ist durch:
ein bis drei Halogenatome
eine Cyangruppe,
eine C₁-C₄Alkoxygruppe,
eine C₁-C₆Alkylcarbonyloxygruppe,
eine Phenylcarbonyloxygruppe, die gegebenenfalls durch ein bis drei Halogenatome oder eine C₁-C₄Alkylgruppe substituiert ist, oder
eine Benzylcarbonyloxygruppe; und
R₁ Phenyl bedeutet, das gegebenenfalls durch ein bis drei Halogenatome, eine oder zwei C₁-C₄Alkylgruppen, eine oder zwei C₁-C₄Alkoxygruppen, eine CN-Gruppe, eine NO₂-Gruppe oder eine CF₃-Gruppe substituiert ist.

3. Verbindung nach Anspruch 2, die aus der Gruppe
2-(*p*-Chlorphenyl)-4-[(3,4-dichlorphenyl)thio]-5-(trifluormethyl)pyrrol-3-carbonitril;
2-(*p*-Chlorphenyl)-4-[(*p*-chlorphenyl)thio]-5-(trifluormethyl)pyrrol-3-carbonitril;
3-Brom-4-[(3-chlor-4-fluorphenyl)sulfonyl]-5-(*p*-chlorphenyl)-2-(trifluormethyl)pyrrol;
3-Brom-5-(*p*-chlorphenyl)-4-[(*p*-chlorphenyl)sulfonyl]-2-(trifluormethyl)pyrrol;
4-Brom-2-(*p*-chlorphenyl)-5-[(*p*-chlorphenyl)sulfonyl]pyrrol-3-carbonitril;
2-(*p*-Chlorphenyl)-5-[(3,4-dichlorphenyl)sulfonyl]pyrrol-3-carbonitril; und
2-(*p*-Chlorphenyl)-5-[(3,4-dichlorphenyl)sulfonyl]-1-(ethoxymethyl)pyrrol-3-carbonitril stammt.

4. Verfahren zur Bekämpfung von Insekten, **dadurch gekennzeichnet, daß** man die Insekten, ihre Vermehrungsstätten, ihre Nahrung oder ihre Umgebung mit einer insektizid wirksamen Menge einer Verbindung der Strukturformel 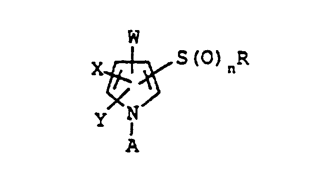 in der A, R, W, X, Y und n wie in Anspruch 1 beschrieben sind,
in Kontakt bringt.

5. Verfahren nach Anspruch 4, bei dem die Verbindung aus der Gruppe
2-(*p*-Chlorphenyl)-4-[(3,4-dichlorphenyl)thio]-5-(trifluormethyl)pyrrol-3-carbonitril;
2-(*p*-Chlorphenyl)-4-[(*p*-chlorphenyl)thio]-5-(trifluormethyl)pyrrol-3-carbonitril;
3-Brom-4-[(3-chlor-4-fluorphenyl)sulfonyl]-5-(*p*-chlorphenyl)-2-(trifluormethyl)pyrrol;
3-Brom-5-(*p*-chlorphenyl)-4-[(*p*-chlorphenyl)sulfonyl]-2-(trifluormethyl)pyrrol;
4-Brom-2-(*p*-chlorphenyl)-5-[(*p*-chlorphenyl)sulfonyl]pyrrol-3-carbonitril;
2-(*p*-Chlorphenyl)-5-[(3,4-dichlorphenyl)sulfonyl]pyrrol-3-carbonitril; und
2-(*p*-Chlorphenyl)-5-[(3,4-dichlorphenyl)sulfonyl]-1-(ethoxymethyl)pyrrol-3-carbonitril stammt.

6. Verfahren zum Schutz von wachsenden Pflanzen gegen Insektenbefall, **dadurch gekennzeichnet, daß** man auf das Blattwerk der Pflanzen oder auf den Boden oder das Wasser, in dem sie wachsen, eine insektizid wirksame Menge einer Verbindung der Strukturformel 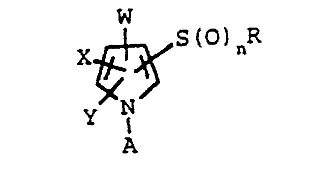 in der A, R, W, X, Y und n wie in Anspruch 1 beschrieben sind,
ausbringt.

7. Verfahren nach Anspruch 6, bei dem die Verbindung aus der Gruppe
2-(*p*-Chlorphenyl)-4-[(3,4-dichlorphenyl)thio]-5-(trifluormethyl)pyrrol-3-carbonitril;
2-(*p*-Chlorphenyl)-4-[(*p*-chlorphenyl)thio]-5-(trifluormethyl)pyrrol-3-carbonitril;
3-Brom-4-[(3-chlor-4-fluorphenyl)sulfonyl]-5-(*p*-chlorphenyl)-2-(trifluormethyl)pyrrol;
3-Brom-5-(*p*-chlorphenyl)-4-[(*p*-chlorphenyl)sulfonyl]-2-(trifluormethyl)pyrrol;
4-Brom-2-(*p*-chlorphenyl)-5-[(*p*-chlorphenyl)sulfonyl]pyrrol-3-carbonitril;
2-(*p*-Chlorphenyl)-5-[(3,4-dichlorphenyl)sulfonyl]pyrrol-3-carbonitril; und
2-(*p*-Chlorphenyl)-5-[(3,4-dichlorphenyl)sulfonyl]-1-(ethoxymethyl)pyrrol-3-carbonitril stammt.

8. Verfahren nach Anspruch 6, wobei man die Verbindung auf die Pflanzen oder den Boden oder das Wasser, in dem sie wachsen, in einer Menge von ungefähr 0,1 kg/ha bis 4,0 kg/ha ausbringt.

9. Mittel zur Bekämpfung von Insekten mit einem landwirtschaftlich unbedenklichen Träger und einer insektizidwirksamen Menge einer Verbindung der Strukturformel 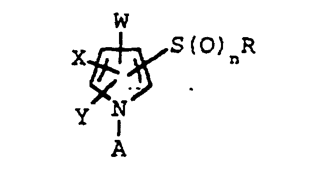 in der A, R, W, X, Y und n wie in Anspruch 1 beschrieben sind.

10. Mittel nach Anspruch 9, bei dem A, R, W, X, Y und n wie in Anspruch 2 beschrieben sind.

11. Verfahren zur Herstellung einer Verbindung der Formel I nach Anspruch 1, **gekennzeichnet durch** eine der folgenden Vorgehensweisen:
a) Umsetzen einer Verbindung der Formel 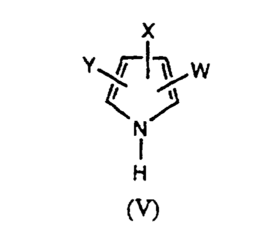 in der W, X und Y wie in Anspruch 1 definiert sind, mit einer Verbindung der Formel
**RSZ** (V1),
in der R wie in Anspruch 1 definiert ist und Z Cl oder Br bedeutet, so daß man eine Verbindung der Formel I erhält, in der A Wasserstoff und n 0 bedeutet, sowie gewünschtenfalls Oxidation mit einem Oxidationsmittel, so daß man eine entsprechende Verbindung der Formel I erhält, in der n 1 oder 2 bedeutet; oder
b) Umsetzen einer Verbindung der Formel I, in der A Wasserstoff darstellt, mit einem Alkylierungsmittel, Alkenylierungsmittel oder Alkinylierungsmittel der Formel
**A-Halogenid,**
in der A C₁-C₆Alkyl, C₃-C₆Alkenyl oder C₃-C₆Alkinyl bedeutet, die jeweils wie in Anspruch 1 definiert substituiert sind, so daß man eine entsprechende Verbindung der Formel I erhält;
oder
c) Acylierung einer Verbindung der Formel I, die wie in Anspruch 1 definiert ist und in der A Wasserstoff bedeutet, mit einem Acylchlorid der Formel
**R**_{**1**}**COCl**
in der R₁ wie in Anspruch 1 definiert ist, so daß man eine entsprechende Verbindung der Formel I erhält, in der A COR₁ bedeutet;
oder
d) Umsetzen einer Verbindung der Formel I, die wie in Anspruch 1 definiert ist und in der A CH₂Cl bedeutet, mit einer Verbindung der Formel
**(Alkali)-SQ,**
in der Q wie in Anspruch 1 definiert ist, so daß man eine entsprechende Verbindung der Formel I erhält, in der A SQ bedeutet;
oder
e) Umsetzen einer Verbindung der Formel I, die wie in Anspruch 1 definiert ist und in der A H bedeutet, mit einer Verbindung der Formel
**R**_{**3**}**C(O)NHCH(R**_{**2**}**)OC(O)CH**_{**3**}**,**
in der R₂ und R₃ wie in Anspruch 1 definiert sind, so daß man eine entsprechende Verbindung der Formel I erhält, in der A CHR₂NHC(O)R₃ bedeutet;
oder
f) Umsetzen einer Verbindung der Formel I, die wie in Anspruch 1 definiert ist und in der A CHR₂NHC(O)R₃ bedeutet, mit einem Phosphoroxyhalogenid (wie zum Beispiel POCl₃), so daß man eine entsprechende Verbindung der Formel I erhält, in der A -CHR₂-Halogen darstellt (d.h. falls R₂ = Wasserstoff, dann A = Halogenmethyl und falls R₂ = C₁-C₄Alkyl, dann bedeutet A α-Halogen(C₂-C₅)alkyl);
oder
g) Umsetzen einer Verbindung der Formel I, die wie in Anspruch 1 definiert ist und in der A -CHR₂-Halogen darstellt, mit einer Verbindung der Formel
**Q**_{**1**}**(CR**_{**5**}**R**_{**6**}**)**_{**m**}**CO**_{**2**}**H,**
in der m, R₅, R₆ und Q wie in Anspruch 1 definiert sind, so daß man eine entsprechende Verbindung der Formel I erhält, in der A
**-CHR**_{**4**}**OC(O)(CR**_{**5**}**R**_{**6**}**)**_{**m**}**Q**_{**1**}**,**
wie in Anspruch 1 definiert bedeutet.

## Revendications

1. Composé ayant la formule développée 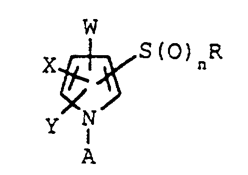 dans laquelle
R et X sont chacun indépendamment un groupe phényle facultativement substitué par toute association de un à cinq atomes d'halogène ou groupes NO₂, CN, alkyle en C₁-C₄, halogénoalkyle en C₁-C₄, alcoxy en C₁-C₄ ou halogénoalcoxy en C₁-C₄ ;
n est le nombre entier 0, 1 ou 2 ;
W est un halogène, CN, NO₂ ou un groupe halogénoalkyle en C₁-C₄
Y est l'hydrogène, un halogène ou un groupe halogénoalkyle en C₁-C₄ ;
A est l'hydrogène, CN, C(O)R₁, CHR₂NHC(O)R₃, CH₂SQ,
CHR₄OC(O)(CR₅R₆)ₘQ₁, un groupe alkyle en C₁-C₆ facultativement substitué par
un à trois atomes d'halogène,
un groupe tri(alkyle en C₁-C₄)silyle,
un groupe hydroxyle,
un groupe cyano,
un ou deux groupes alcoxy en C₁-C₄ facultativement substitués par un à trois atomes d'halogène,
un groupe alkylthio en C₁-C₄,
un groupe phényle facultativement substitué par un à trois substituants, identiques ou différents, choisis parmi les atomes d'halogène et les groupes alkyle en C₁-C₄ et alcoxy en C₁-C₄,
un groupe phénoxy facultativement substitué par un à trois substituants, identiques ou différents, choisis parmi les atomes d'halogène et les groupes alkyle en C₁-C₄ et alcoxy en C₁-C₄,
un groupe benzyloxy facultativement substitué sur le noyau phénylique par un à trois substituants, identiques ou différents, choisis parmi les atomes d'halogène et les groupes alkyle en C₁-C₄ et alcoxy en C₁-C₄,
un groupe (alkyle en C₁-C₆)carbonyloxy facultativement substitué par un à trois atomes d'halogène,
un groupe (alcényle en C₂-C₆)carbonyloxy facultativement substitué par un à trois atomes d'halogène,
un groupe phénylcarbonyloxy facultativement substitué par un à trois substituants, identiques ou différents, choisis parmi les atomes d'halogène et les groupes alkyle en C₁-C₄ et alcoxy en C₁-C₄,
un groupe (alcoxy en C₁-C₆)carbonyle facultativement substitué par un à trois atomes d'halogène ou un à trois groupes alcoxy en C₁-C₄, ou
un groupe benzylcarbonyloxy facultativement substitué sur le noyau phénylique par un à trois substituants, identiques ou différents, choisis parmi les atomes d'halogène et les groupes alkyle en C₁-C₄ et alcoxy en C₁-C₄,
un groupe alcényle en C₃-C₆ facultativement substitué par un à trois atomes d'halogène ou par un groupe phényle, ou
un groupe alcynyle en C₃-C₆ facultativement substitué par un à trois atomes d'halogène ou par un groupe phényle ;
R₁ est un groupe alkyle en C₁-C₆ ou cycloalkyle en C₃-C₆,
chacun étant facultativement substitué par
un à trois atomes d'halogène,
un groupe hydroxyle,
un groupe cyano,
un ou deux groupes alcoxy en C₁-C₄ facultativement substitués par un à trois atomes d'halogène,
un groupe alkylthio en C₁-C₄,
un groupe phényle facultativement substitué par un à trois substituants, identiques ou différents, choisis parmi les atomes d'halogène et les groupes alkyle en C₁-C₄ et alcoxy en C₁-C₄,
un groupe phénoxy facultativement substitué par un à trois substituants, identiques ou différents, choisis parmi les atomes d'halogène et les groupes alkyle en C₁-C₄ et alcoxy en C₁-C₄,
un groupe benzyloxy facultativement substitué sur le noyau phénylique par un à trois substituants, identiques ou différents, choisis parmi les atomes d'halogène et les groupes alkyle en C₁-C₄ et alcoxy en C₁-C₄,
un groupe (alkyle en C₁-C₆)carbonyloxy facultativement substitué par un à trois atomes d'halogène,
un groupe (alcényle en C₂-C₆)carbonyloxy facultativement substitué par un à trois atomes d'halogène,
un groupe phénylcarbonyloxy facultativement substitué par un à trois substituants, identiques ou différents, choisis parmi les atomes d'halogène et les groupes alkyle en C₁-C₄ et alcoxy en C₁-C₄,
un groupe (alcoxy en C₁-C₆)carbonyle facultativement substitué par un à trois atomes d'halogène ou un à trois groupes alcoxy en C₁-C₄, ou
un groupe benzylcarbonyloxy facultativement substitué sur le noyau phénylique par un à trois substituants, identiques ou différents, choisis parmi les atomes d'halogène et les groupes alkyle en C₁-C₄ et alcoxy en C₁-C₄,
un groupe alcényle en C₂-C₆ facultativement substitué par un à trois atomes d'halogène ou par un groupe phényle,
un groupe alcynyle en C₃-C₆ facultativement substitué par un à trois atomes d'halogène ou par un groupe phényle,
un groupe phényle facultativement substitué par un ou plusieurs substituants, identiques ou différents, choisis parmi les atomes d'halogène et les groupes alkyle en C₁-C₄, alcoxy en C₁-C₄, phénoxy, alkylthio en C₁-C₄, tri(alkyle en C₁-C₄)silyle, alkylsulfinyle en C₁-C₄, alkylsulfonyle en C₁-C₄, CN, NO₂ et CF₃,
un groupe phénoxy facultativement substitué par un ou plusieurs substituants, identiques ou différents, choisis parmi les atomes d'halogène et les groupes alkyle en C₁-C₄, alcoxy en C₁-C₄, alkylthio en C₁-C₄, tri(alkyle en C₁-C₄)silyle, alkylsulfinyle en C₁-C₄, alkylsulfonyle en C₁-C₄, CN, NO₂ et CF₃,
un groupe 1- ou 2-naphtyle,
un groupe 2-, 3- ou 4-pyridyle facultativement substitué par un à trois atomes d'halogène,
un groupe alcoxy en C₁-C₆ facultativement substitué par un à trois atomes d'halogène, ou
un groupe alcényloxy en C₂-C₆ facultativement substitué par un à trois atomes d'halogène ;
R₂ est l'hydrogène ou un groupe alkyle en C₁-C₄ ;
R₃ est un groupe alkyle en C₁-C₆ facultativement substitué par un à trois atomes d'halogène,
un groupe phényle facultativement substitué par un à trois substituants, identiques ou différents, choisis parmi les atomes d'halogène et les groupes CN, NO₂, alkyle en C₁-C₄, alcoxy en C₁-C₄ et CF₃,
un groupe 2- ou 3-thiényle,
un groupe 2- ou 3-furyle ;
Q est 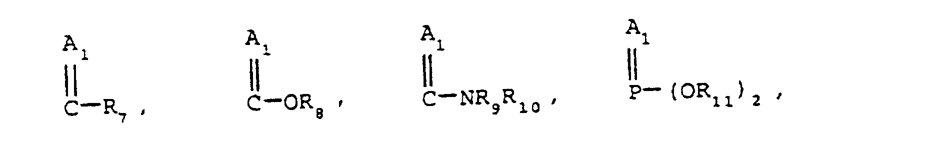 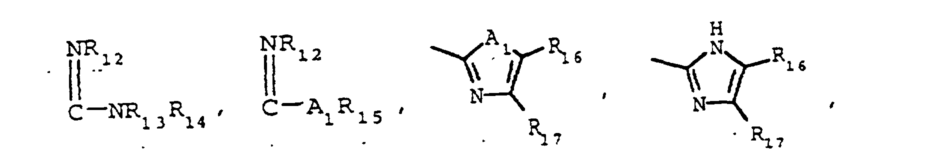 CN,
un groupe alkyle en C₁-C₆ facultativement substitué par un ou plusieurs atomes d'halogène, groupes CN ou groupes phényle, ou
un groupe phényle facultativement substitué par un ou plusieurs substituants, identiques ou différents, choisis parmi les atomes d'halogène et les groupes alkyle en C₁-C₄, alcoxy en C₁-C₄, CN, NO₂, CF₃ et NR₁₈R₁₉ ;
A₁ est O ou S ;
R₇ est un groupe alkyle en C₁-C₆ ou phényle ;
R₈ est un groupe alkyle en C₁-C₆ ;
R₉ et R₁₀ sont chacun indépendamment l'hydrogène, un groupe alkyle en C₁-C₆, ou peuvent être pris avec l'atome auquel ils sont liés pour former un hétérocycle ayant 5 à 7 chaînons ;
R₁₁ est un groupe alkyle en C₁-C₄ ;
R₁₂ est l'hydrogène, un groupe alkyle en C₁-C₄, ou peut être pris avec R₁₃ ou R₁₅ et les atomes auxquels ils sont liés pour former un hétérocycle ayant 5 à 7 chaînons facultativement substitué par un ou deux groupes alkyle en C₁-C₄ ;
R₁₃ et R₁₄ sont chacun indépendamment l'hydrogène ou un groupe alkyle en C₁-C₄ ;
R₁₅ est un groupe alkyle en C₁-C₄ ou, lorsqu'il est pris avec R₁₂ et les atomes auxquels ils sont liés, peut former un hétérocycle ayant 5 à 7 chaînons facultativement substitué par un ou deux groupes alkyle en C₁-C₄ ;
R₁₆ et R₁₇ sont chacun indépendamment l'hydrogène ou un groupe alkyle en C₁-C₄ ou, lorsqu'ils sont pris ensemble, peuvent former un cycle dans lequel R₁₆R₁₇ représente -CH=CH-CH=CH- ;
R₁₈ et R₁₉ sont chacun indépendamment l'hydrogène ou un groupe alkyle en C₁-C₄ ;
R₄ est l'hydrogène ou un groupe alkyle en C₁ - C₄ ;
R₅ et R₆ sont chacun indépendamment l'hydrogène,
un groupe alkyle en C₁-C₆ facultativement substitué par un ou plusieurs atomes d'halogène,
un groupe alcoxy en C₁-C₆ facultativement substitué par un ou plusieurs atomes d'halogène,
un groupe alkylthio en C₁-C₆ facultativement substitué par un ou plusieurs atomes d'halogène, ou
un groupe phényle facultativement substitué par un ou plusieurs
atomes d'halogène,
groupes NO₂ ;
groupes CN,
groupes alkyle en C₁-C₄ facultativement substitués par un ou plusieurs atomes d'halogène ou
groupes alcoxy en C₁-C₄ facultativement substitués par un ou plusieurs atomes d'halogène, ou
lorsque R₅ et R₆ sont pris avec l'atome auquel ils sont liés, ils peuvent former un groupe cycloalkyle en C₃-C₆ facultativement substitué par un à trois groupes alkyle en C₁-C₄, groupes alcényle en C₂-C₆ ou groupes phényle, ou bien R₅ et R₆ peuvent être pris avec R₂₀ et les atomes auxquels ils sont liés pour former un hétérocycle ayant 4 à 7 chaînons ;
m est le nombre entier 0, 1, 2, 3 ou 4 ;
Q₁ est A₂R₂₀, 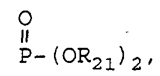 NR₂₂R₂₃, CR₂₄R₂₅C(O)R₂₆, ou
un groupe cycloalkyle en C₃-C₆ facultativement substitué par un ou plusieurs groupes alkyle en C₁-C₆, groupes alcényle en C₂-C₆, ou
groupes phényle facultativement substitués par un ou plusieurs substituants, identiques ou différents, choisis parmi
des atomes d'halogène,
des groupes NO₂,
des groupes CN,
des groupes alkyle en C₁-C₄ facultativement substitué par un ou plusieurs atomes d'halogène, ou
des groupes alcoxy en C₁-C₄ facultativement substitués par un ou plusieurs atomes d'halogène ;
A₂ est O ou S(O)ₚ ;
p est le nombre entier 0, 1 ou 2 ;
R₂₀ est l'hydrogène,
un groupe alkyle en C₁-C₆,
un groupe alcényle en C₂-C₆,
un groupe alcynyle en C₂-C₆,
un groupe phényle facultativement substitué par un ou plusieurs substituants, identiques ou différents, choisis parmi
des atomes d'halogène,
des groupes NO₂,
des groupes CN,
des groupes alkyle en C₁-C₄ facultativement substitués par un ou plusieurs atomes d'halogène,
des groupes alcoxy en C₁-C₄ facultativement substitués par un ou plusieurs atomes d'halogène,
C(O)R₂₇ à condition que p soit 0,
C(O)R₂₈ à condition que p soit 0,
(CH₂CH₂O)_{q}R₂₇, ou 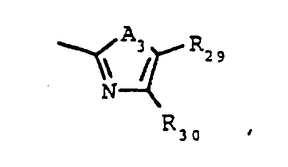 ou bien
R₂₀ peut être pris avec R₅ ou avec R₆ et les atomes auxquels ils sont liés pour former un hétérocycle ayant 4 à 7 chaînons ;
A₃ est O ou S ;
R₂₇ est un groupe alkyle en C₁-C₆,
un groupe alcényle en C₂-C₆,
un groupe alcynyle en C₂-C₆, ou
un groupe phényle facultativement substitué par un ou plusieurs substituants, identiques ou différents, choisis parmi
des atomes d'halogène,
des groupes NO₂,
des groupes CN,
des groupes alkyle en C₁-C₄ facultativement substitués par un ou plusieurs atomes d'halogène, ou
des groupes alcoxy en C₁-C₄ facultativement substitués par un ou plusieurs atomes d'halogène ;
q est le nombre entier 1, 2 ou 3 ;
R₂₈ est OR₃₁ ou NR₃₂R₃₃ ;
R₃₁ est un groupe alkyle en C₁-C₆ ou
un groupe phényle facultativement substitué par un ou plusieurs substituants, identiques ou différents, choisis parmi
des atomes d'halogène,
des groupes NO₂,
des groupes CN,
des groupes alkyle en C₁-C₄ facultativement substitués par un ou plusieurs atomes d'halogène, ou
des groupes alcoxy en C₁-C₄ facultativement substitués par un ou plusieurs atomes d'halogène ;
R₃₂ et R₃₃ sont chacun indépendamment l'hydrogène ou un groupe alkyle en C₁-C₄ ;
R₂₉ et R₃₀ sont chacun indépendamment l'hydrogène ou un groupe alkyle en C₁-C₄ ou, lorsqu'ils sont pris ensemble, peuvent former un cycle dans lequel R₂₉R₃₀ représente -CH=CH-CH=CH- ;
R₂₁ est un groupe alkyle en C₁-C₄ ;
R₂₂ est l'hydrogène,
un groupe alkyle en C₁-C₆,
un groupe alcényle en C₂-C₆,
alcynyle en C₂-C₆, ou
phényle facultativement substitué par un ou plusieurs substituants, identiques ou différents, choisis parmi des atomes d'halogène,
des groupes NO₂,
des groupes CN,
des groupes alkyle en C₁-C₄ facultativement substitués par un ou plusieurs atomes d'halogène, ou
des groupes alcoxy en C₁-C₄ facultativement substitués par un ou plusieurs atomes d'halogène, ou bien
R₂₂ peut être pris avec R₅ ou avec R₆ et les atomes auxquels ils sont liés pour former un hëtérocycle ayant 4 à 7 chaînons ;
R₂₃ est l'hydrogène,
un groupe alkyle en C₁-C₆,
un groupe alcényle en C₂-C₆,
un groupe alcynyle en C₂-C₆,
un groupe phényle facultativement substitué par un ou plusieurs substituants, identiques ou différents, choisis parmi
des atomes d'halogène,
des groupes NO₂,
des groupes CN,
des groupes alkyle en C₁-C₄ facultativement substitués par un ou plusieurs atomes d'halogène, ou
des groupes alcoxy en C₁-C₄ facultativement substitués par un ou plusieurs atomes d'halogène ;
C(A₄)R₃₄,
CN,
SO₂R₃₅, ou
C(O)CHR₃₆NHR₃₇ ;
A₄ est O ou S ;
R₃₄ est OR₃₈, CO₂R₃₈, NR₃₉R₄₀,
un groupe alkyle en C₁-C₆ facultativement substitué par un à trois atomes d'halogène,
un groupe alcényle en C₂-C₆,
un groupe alcynyle en C₂-C₆, ou
un groupe phényle facultativement substitué par un ou plusieurs substituants, identiques ou différents, choisis parmi
des atomes d'halogène,
des groupes NO₂,
des groupes CN,
des groupes alkyle en C₁-C₄ facultativement substitués par un ou plusieurs atomes d'halogène, ou
des groupes alcoxy en C₁-C₄ facultativement substitués par un ou plusieurs atomes d'halogène ;
R₃₈ est un groupe alkyle en C₁-C₆ facultativement substitué par un groupe phényle, ou
un groupe phényle facultativement substitué par un ou plusieurs substituants, identiques ou différents, choisis parmi
des atomes d'halogène,
des groupes NO₂,
des groupes CN,
des groupes alkyle en C₁-C₄ facultativement substitués par un ou plusieurs atomes d'halogène, ou
des groupes alcoxy en C₁-C₄ facultativement substitués par un ou plusieurs atomes d'halogène ;
R₃₉ et R₄₀ sont chacun indépendamment l'hydrogène ou un groupe alkyle en C₁-C₄ ;
R₃₅ est NR₄₁R₄₂,
un groupe alkyle en C₁-C₆,
un groupe alcényle en C₂-C₆,
un groupe alcynyle en C₂-C₆, ou
un groupe phényle facultativement substitué par un ou plusieurs substituants, identiques ou différents, choisis parmi
des atomes d'halogène,
des groupes NO₂,
des groupes CN,
des groupes alkyle en C₁-C₄ facultativement substitués par un ou plusieurs atomes d'halogène, ou
des groupes alcoxy en C₁-C₄ facultativement substitués par un ou plusieurs atomes d'halogène ;
R₄₁ et R₄₂ sont chacun indépendamment l'hydrogène ou un groupe alkyle en C₁-C₄ ;
R₃₆ est l'hydrogène,
un groupe alkyle en C₁-C₄ facultativement substitué par
un groupe hydroxyle,
un groupe SR₄₃,
un groupe C(O)NH₂,
un groupe NH₂,
un groupe NHC(=NH)NH₂,
un groupe CO₂H,
un groupe phényle facultativement substitué par un groupe hydroxyle,
un groupe 3-indolyle ou
un groupe 4-imidazolyle ;
R₄₃ est l'hydrogène ou un groupe alkyle en C₁-C₄ ;
R₃₇ est C(A₄)R₄₄ ;
R₄₄ est un groupe alkyle en C₁-C₆ facultativement substitué par un ou plusieurs atomes d'halogène,
un groupe alcoxyalkyle en C₂-C₆,
un groupe alkylthio en C₁-C₆,
un groupe phényle facultativement substitué par un ou plusieurs substituants, identiques ou différents, choisis parmi
des atomes d'halogène,
des groupes NO₂,
des groupes CN,
des groupes alkyle en C₁-C₄ facultativement substitués par un ou plusieurs atomes d'halogène, ou
des groupes alcoxy en C₁-C₄ facultativement substitués par un ou plusieurs atomes d'halogène ;
OR₃₈,
CO₂R₃₈ ou
NR₃₉R₄₀ ;
R₂₄ et R₂₅ sont chacun indépendamment l'hydrogène,
un groupe alkyle en C₁-C₆ facultativement substitué par un plusieurs atomes d'halogène,
un groupe alcoxy en C₁-C₆ facultativement substitué par un ou plusieurs atomes d'halogène,
un groupe alkylthio en C₁-C₆ facultativement substitué par un ou plusieurs atomes d'halogène,
un groupe phényle facultativement substitué par un ou plusieurs substituants, identiques ou différents, choisis parmi
des atomes d'halogène,
des groupes CN,
des groupes NO₂,
des groupes alkyle en C₁-C₄ facultativement substitués par un ou plusieurs atomes d'halogène, ou
des groupes alcoxy en C₁-C₄ facultativement substitués par un ou plusieurs atomes d'halogène ; ou bien
lorsque R₂₄ et R₂₅ sont pris avec l'atome auquel ils sont liés, ils peuvent former un groupe cycloalkyle en C₃-C₆ facultativement substitué par un à trois groupes alkyle en C₁-C₄, groupes alcényle en C₂-C₆ ou groupes phényle ;
R₂₆ est OR₄₅, NR₄₁R₄₂, un groupe alkyle en C₁-C₄, ou
un groupe phényle facultativement substitué par un ou plusieurs substituants, identiques ou différents, choisis parmi
des atomes d'halogène,
des groupes CN,
des groupes NO₂,
des groupes alkyle en C₁-C₄ facultativement substitués par un ou plusieurs atomes d'halogène, ou
des groupes alcoxy en C₁-C₄ facultativement substitués par un ou plusieurs atomes d'halogène ; et
R₄₅ est un groupe alkyle en C₁-C₄ ou
un groupe phényle facultativement substitué par un ou plusieurs substituants, identiques ou différents, choisis parmi
des atomes d'halogène,
des groupes CN,
des groupes NO₂,
des groupes alkyle en C₁-C₄ facultativement substitués par un ou plusieurs atomes d'halogène, ou
des groupes alcoxy en C₁-C₄ facultativement substitués par un ou plusieurs atomes d'halogène.

2. Composé selon la revendication 1, dans lequel
R et X sont chacun indépendamment un groupe phényle facultativement substitué par toute association d'un à trois atomes d'halogène ou groupes NO₂, CN, alkyle en C₁-C₄, halogénoalkyle en C₁-C₄, alcoxy en C₁-C₄ ou halogénoalcoxy en C₁-C₄ ;
n est le nombre entier 0, 1 ou 2 ;
W est Cl, Br, CN, NO₂ ou un groupe halogénoalkyle en C₁-C₄ ;
Y est l'hydrogène, Cl, Br ou un groupe halogénoalkyle en C₁-C₄ ;
A est l'hydrogène, CN, C(O)R₁ ou
un groupe alkyle en C₁-C₄ facultativement substitué par
un à trois atomes d'halogène,
un groupe cyano,
un groupe alcoxy en C₁-C₄,
un groupe (alkyle en C₁-C₆)carbonyloxy,
un groupe phénylcarbonyloxy facultativement substitué par un à trois atomes d'halogène ou par un groupe alkyle en C₁-C₄, ou
un groupe benzylcarbonyloxy ; et
R₁ est un groupe phényle facultativement substitué par un à trois atomes d'halogène, un ou deux groupes alkyle en C₁-C₄, un ou deux groupes alcoxy en C₁-C₄, un groupe CN, un groupe NO₂ ou un groupe CF₃.

3. Composé selon la revendication 2, choisi dans la classe formée par
le 2-(*p*-chlorophényl)-4-[(3,4-dichlorophényl)thio]-5-(trifluorométhyl)pyrrole-3-carbonitrile ;
le 2-(*p*-chlorophényl)-4-[(*p*-chlorophényl)thio]-5-(trifluorométhyl)pyrrole-3-carbonitrile ;
le 3-bromo-4-[(3-chloro-4-fluorophényl)sulfonyl]-5-(*p*-chlorophényl)-2-(trifluorométhyl)pyrrole ;
le3-bromo-5-(*p*-chlorophényl)-4-[(*p*-chlorophényl)sulfonyl]-2-(trifluorométhyl)pyrrole ;
le4-bromo-2-(*p*-chlorophényl)-5-[(*p*-chlorophényl)sulfonyl]-pyrrole-3-carbonitrile ;
le 2-(*p*-chlorophényl)-5-[(3,4-dichlorophényl) sulfonyl]-pyrrole-3-carbonitrile ; et
le 2-(*p*-chlorophényl)-5-[(3,4-dichlorophényl)sulfonyl]-1-(éthoxyméthyl)pyrrole-3-carbonitrile.

4. Procédé de lutte contre des insectes, qui consiste à mettre lesdits insectes, leurs zones de reproduction, leur source de nourriture ou leur habitat, avec une quantité à effet insecticide d'un composé ayant la formule développée 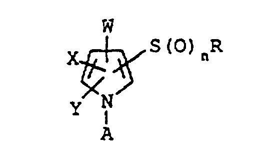 dans laquelle A, R, W, X, Y et n sont tels que définis dans la revendication 1.

5. Procédé selon la revendication 4, dans lequel le composé est choisi dans la classe formée par
le 2-(*p*-chlorophényl)-4-[(3,4-dichlorophényl)thio]-5-(trifluorométhyl)pyrrole-3-carbonitrile ;
le 2-(*p*-chlorophényl)-4-[(*p*-chlorophényl)thio]-5-(trifluorométhyl)pyrrole-3-carbonitrile ;
le 3-bromo-4-[(3-chloro-4-fluorophényl)sulfonyl]-5-(*p*-chlorophényl)-2-(trifluorométhyl)pyrrole ;
le3-bromo-5-(*p*-chlorophényl)-4-[(*p*-chlorophényl)sulfonyl]-2-(trifluorométhyl)pyrrole ;
le4-bromo-2-(*p*-chlorophényl)-5-[(*p*-chlorophényl)sulfonyl]-pyrrole-3-carbonitrile ;
le 2-(*p*-chlorophényl)-5-[(3,4-dichlorophényl)sulfonyl]-pyrrole-3-carbonitrile ; et
le 2-(*p*-chlorophényl)-5-(3,4-dichlorophényl)sulfonyl]-1-(éthoxyméthyl)pyrrole-3-carbonitrile.

6. Procédé de protection de plantes en croissance contre une attaque par des insectes, qui consiste à appliquer au feuillage desdites plantes ou à la terre ou à l'eau dans laquelle elles poussent une quantité à effet insecticide d'un composé ayant la formule développée 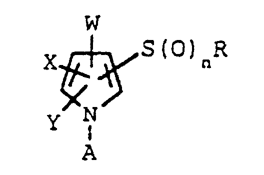 dans laquelle A, R, W, X, Y et n sont tels que définis dans la revendication 1.

7. Procédé selon la revendication 6, dans lequel le composé est choisi dans la classe formée par
le 2-(*p*-chlorophényl)-4-[(3,4-dichlorophényl)thio]-5-(trifluorométhyl)pyrrole-3-carbonitrile ;
le 2-(*p*-chlorophényl)-4-[(*p-*chlorophényl)thio]-5-(trifluorométhyl)pyrrole-3-carbonitrile ;
le 3-bromo-4-[(3-chloro-4-fluorophényl)sulfonyl]-5-(*p*-chlorophényl)-2-(trifluorométhyl)pyrrole ;
le3-bromo-5-(*p*-chlorophényl)-4-[(*p*-chlorophényl)sulfonyl]-2-(trifluorométhyl)pyrrole ;
le4-bromo-2-(*p*-chlorophényl)-5-[(*p*-chlorophényl)sulfonyl]-pyrrole-3-carbonitrile ;
le 2-(*p*-chlorophényl)-5-[(3,4-dichlorophényl)sulfonyl]-pyrrole-3-carbonitrile ; et
le2-(*p*-chlorophényl)-5-[(3,4-dichlorophényl)sulfonyl]-1-(éthoxyméthyl)pyrrole-3-carbonitrile.

8. Procédé selon la revendication 6, dans lequel le composé est appliqué aux plantes ou à la terre ou à l'eau dans laquelle elles poussent à une dose d'environ 0,1 kg/ha à 4,0 kg/ha.

9. Composition destinée à combattre des insectes, qui comprend un support acceptable en usage agronomique et une quantité à effet insecticide d'un composé ayant la formule développée 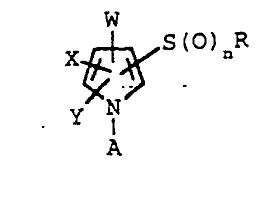 dans laquelle A, R, W, X, Y et n sont tels que définis dans la revendication 1.

10. Composition selon la revendication 9, dans laquelle A, R, W, X, Y et n sont tels que définis dans la revendication 2.

11. Procédé pour préparer un composé de formule I tel que défini dans la revendication 1, qui comprend l'une des étapes suivantes :
a) faire réagir un composé de formule 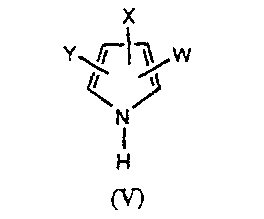 où W, X et Y sont tels que définis dans la revendication 1, avec un composé de formule
RSZ (VI)
où R est tel que défini dans la revendication 1 et Z est Cl ou Br, pour obtenir un composé de formule I dans lequel A est l'hydrogène et n est 0, et facultativement oxyder avec un agent oxydant pour obtenir un composé correspondant de formule I dans lequel n est 1 ou 2 ;
ou
b) faire réagir un composé de formule I dans lequel A est l'hydrogène avec un agent alkylant, alcénylant, alcynylant de formule :
A-halogénure
où A est un groupe alkyle en C₁-C₆, alcényle en C₃-C₆ ou alcynyle en C₃-C₆, chacun étant facultativement substitué comme défini dans la revendication 1, pour obtenir un composé correspondant de formule I ;
ou
c) acyler un composé de formule I tel que défini dans la revendication 1, dans lequel A est l'hydrogène, avec un chlorure d'acyle de formule
R₁COCl
où R₁ est tel que défini dans la revendication 1, pour obtenir un composé correspondant de formule I dans lequel A est COR₁ ;
ou
d) faire réagir un composé de formule I tel que défini dans la revendication 1, dans lequel A est CH₂Cl, avec un composé de formule
(métal alcalin)SQ
où Q est tel que défini dans la revendication 1, pour obtenir un composé correspondant de formule I dans lequel A est SQ ;
ou
e) faire réagir un composé de formule I tel que défini dans la revendication 1, dans lequel A est H, avec un composé de formule
R₃C(O)NHCH(R₂)OC(O)CH₃
où R₂ et R₃ sont tels que définis dans la revendication 1, pour obtenir un composé correspondant de formule I dans lequel A est CHR₂NHC(O)R₃ ;
ou
f) faire réagir un composé de formule I tel que défini dans la revendication 1, dans lequel A est CHR₂NHC(O)R₃, avec un oxyhalogénure de phosphore (tel que POCl₃) pour obtenir un composé correspondant de formule I dans lequel A représente -CHR₂-halogène (c'est-à-dire que, lorsque R₂ = hydrogène, A = halogénométhyle, et lorsque R₂ = alkyle en C₁-C₄, A représente un groupe α-halogéno(alkyle en C₂-C₅)) ;
ou
g) faire réagir un composé de formule I tel que défini dans la revendication 1, dans lequel A est -CHR₂-halogène, avec un composé de formule
Q₁(CR₅R₆)ₘCO₂H
où m, R₅, R₆ et Q sont tels que définis dans la revendication 1, pour obtenir un composé correspondant de formule I dans lequel A est
-CHR₄OC(O) (CR₅R₆)ₘQ₁
tel que défini dans la revendication 1.
